# EUROPEAN PATENT APPLICATION

(11) **EP 3 943 615 A1**
(43) Date of publication of application: **26.01.2022**
(21) Application number: 21184021.0
(22) Date of filing: 27.02.2015
(51) Int. Cl.: C12Q 1/6869, C12Q 1/6809, C12Q 1/6886, G16H 15/00

(54) **METHODS FOR ANALYSIS OF SOMATIC MOBILE ELEMENTS, AND USES THEREOF**

(30) Priority: 27.02.2014 US 201461945791 P
(62) Divisional of application: 15754498.2
(71) Applicant: Jumpcode Genomics, Inc., San Diego, CA 92121 (US)
(72) Inventor: BROWN, Keith, Carlsbad, 92008 (US)
(74) Representative: Avidity IP

(57) **Abstract**

Methods and compositions related to the use of Mobile Element Insertions and their adjacent genomic sequences. Methods using MEIs as markers for cellular proliferation, as targets for pharmaceuticals, as markers for tissue fingerprinting and in related methods and compositions are disclosed herein. Methods and compositions relate to the detection, treatment and ongoing monitoring of cell proliferation events, cancer, and deleterious effects of mobile elements in aging, and to the selection, use and monitoring of the success of treatment regimens to address these conditions.

## Description

### CROSS-REFERENCE

The present application claims the benefit of U.S. Provisional Application Serial No. 61/945,791, filed February 27, 2014, which is incorporated by reference herein in its entirety.

### INCORPORATION BY REFERENCE

All publications, patents, and patent applications mentioned in this specification are herein incorporated by reference to the same extent as if each individual publication, patent, or patent application was specifically and individually indicated to be incorporated by reference in its entirety.

### SUMMARY OF INVENTION

Some embodiments relate to methods for identifying mobile element insertion (MEI) tagged cell proliferation comprising the steps of quantitatively measuring MEI levels at a first MEI insertion site in a first nucleic acid sample, quantitatively measuring MEI levels at a first MEI insertion site in a second nucleic acid sample, and identifying the first MEI insertion site as tagging MEI tagged cell proliferation if MEI levels at a first MEI insertion site in a first nucleic acid sample differ substantially from MEI levels at a first MEI insertion site in a second nucleic acid sample. In some aspects of the methods, the first nucleic acid sample and the second nucleic acid sample comprise substantially similar amounts of nucleic acids. In some aspects of the methods, a control nucleic acid is present at substantially similar amounts in the first nucleic acid sample and the second nucleic acid sample. Some aspects of the methods comprise identifying the sequence adjacent to the first MEI insertion site. Some aspects of the methods comprise selecting a treatment associated with efficacy in addressing a defect in the sequence adjacent to the first MEI insertion site. In some aspects of the methods, the first nucleic acid sample and the second nucleic acid sample are obtained from a common individual at a first time point and a second time point. In some aspects of the methods, the first time point and second time point are separated by a treatment administered to the individual. In some aspects of the methods, the treatment comprises cancer therapy. In some aspects of the methods, the first time point and second time point are separated by at least 6 months. In some aspects of the methods, the first time point and second time point are separated by at least 1 year. In some aspects of the methods, the first time point and second time point are separated by at least 2 years. In some aspects of the methods, the first time point and second time point are separated by at least 5 years. In some aspects of the methods, the first nucleic acid sample and the second nucleic acid sample are extracted from blood. In some aspects of the methods, the first nucleic acid sample and the second nucleic acid sample comprise circulating free nucleic acids. In some aspects of the methods, the first nucleic acid sample and the second nucleic acid sample comprise circulating free genomic DNA. In some aspects of the methods, the first nucleic acid sample is obtained from an individual at a first location and the second nucleic acid sample is obtained from the individual at a second location. In some aspects of the methods, the first location comprises a first cancerous tissue. In some aspects of the methods, the second location comprises healthy tissue. In some aspects of the methods, the second location comprises a second cancerous tissue. In some aspects of the methods, the second cancerous tissue and the first cancerous tissue are derived from a common cancer. Some aspects of the methods comprise generating a report disclosing the MEI levels at a first MEI insertion site in a first nucleic acid sample and the MEI levels at a first MEI insertion site in a second nucleic acid sample. In some aspects of the methods, the report is provided to the individual. In some aspects of the methods, the report is provided to a health care professional. In some aspects of the methods, the report is made confidentially.

Some embodiments relate to Mobile Element Insertion (MEI) monitoring regimens comprising the steps of obtaining genome sequence information from an individual comprising a plurality of MEI insertion borders, reviewing the plurality of MEI insertion borders to identify a border adjacent to an oncogene, and monitoring the quantitative abundance of the MEI border adjacent to the oncogene over time. In some aspects of the methods, the monitoring the quantitative abundance of the MEI border adjacent to the oncogene over time comprises obtaining a first blood sample at a first time point, determining the quantitative abundance of the MEI border in the first blood sample at the first time point, obtaining a second blood sample at a second time point, and determining the quantitative abundance of the MEI border in the second blood sample at the second time point. In some aspects of the methods, the monitoring the quantitative abundance of the MEI border adjacent to the oncogene over time comprises obtaining a first tissue sample at a first time point, determining the quantitative abundance of the MEI border in the first tissue sample at the first time point, obtaining a second tissue sample at a second time point, and determining the quantitative abundance of the MEI border in the second tissue sample at the second time point. In some aspects of the methods, the first tissue sample and the second tissue sample comprise tumor tissue. Some aspects of the methods comprise selecting a treatment to address a cancer related to a defect in the oncogene. Some aspects of the methods comprise administering the treatment to address a cancer related to a defect in the oncogene if the quantitative abundance of the MEI insertion site increases in the sample above a threshold from the first time point to the second time point. In some aspects of the methods, the threshold is a 10% increase. In some aspects of the methods, the threshold is a 20% increase. In some aspects of the methods, the threshold is a 30% increase. In some aspects of the methods, the threshold is a 50% increase. Some aspects of the methods comprise administering a first dosage of the treatment to address a cancer related to a defect in the oncogene prior to a first time point, and increasing the dosage if the quantitative abundance of the MEI insertion site fails to decrease in the sample below a threshold from the first time point to the second time point. In some aspects of the methods, the threshold is 90% of the first time point amount. In some aspects of the methods, the threshold is 80% of the first time point amount. In some aspects of the methods, the threshold is 70% of the first time point amount. In some aspects of the methods, the threshold is 60% of the first time point amount. In some aspects of the methods, the threshold is 50% of the first time point amount. In some aspects of the methods, the threshold is 10% of the first time point amount. In some aspects of the methods, the treatment comprises chemotherapy. In some aspects of the methods, the treatment comprises radiotherapy. In some aspects of the methods, the treatment comprises a pharmaceutical that targets a defect in the sequence adjacent to the MEI insertion. In some aspects of the methods, the treatment comprises a pharmaceutical that targets misregulation of a pathway of which a protein encoded by sequence adjacent to a MEI insertion site participates. In some aspects of the methods, the treatment comprises a nucleic acid that specifically binds the MEI insertion junction. In some aspects of the methods, the nucleic acid comprises a piRNA. In some aspects of the methods, the nucleic acid comprises a siRNA. In some aspects of the methods, the nucleic acid comprises a CRISPR nucleic acid. In some aspects of the methods, the nucleic acid directs methylation of the MEI insertion border.

Some embodiments relate to compositions for the in vivo visualization of cancer tissue comprising a nucleic acid probe spanning an MEI border adjacent to an oncogene, coupled to a detection element. In some aspects of the compositions, the detection element comprises a fluorophore. In some aspects of the compositions, the detection element comprises a photoexcitable moiety. In some aspects of the compositions, the probe traverses cell membranes. In some aspects of the compositions, the probe traverses cell nuclear membranes. In some aspects of the compositions, probe fluorescence is dependent upon probe binding to a target nucleic acid sequence comprising a MEI border adjacent to an oncogene. In some aspects of the compositions, the probe is visualized by a hand-held fluorophore excitation device.

Some embodiments relate to methods for monitoring genomic aging, comprising the steps of quantitatively measuring the number of MEI insertion sites in a first nucleic acid sample at a first time period, quantitatively measuring the number of MEI insertion sites in a first nucleic acid sample at a first time period, and correlating an increase in MEI insertion borders with an increase in genomic aging. In some aspects of the methods, the 10% increase in the number of MEI insertion sites indicates genomic aging. In some aspects of the methods, a 20% increase in the number of MEI insertion sites indicates genomic aging. In some aspects of the methods, a 30% increase in the number of MEI insertion sites indicates genomic aging. In some aspects of the methods, a 50% increase in the number of MEI insertion sites indicates genomic aging. Some aspects of the methods comprise recommending an anti-aging regimen if genomic aging is indicated. In some aspects of the methods, the anti-aging regimen comprises caloric restriction. In some aspects of the methods, the anti-aging regimen comprises administration of an NTHE. In some aspects of the methods, the anti-aging regimen comprises administration of a DNA methylase. In some aspects of the methods, the anti-aging regimen comprises administration of a small regulatory eRNA. In some aspects of the methods, the anti-aging regimen comprises administration of a reverse-transcriptase inhibitor. In some aspects of the methods, the anti-aging regimen comprises administration of a retrovirus inhibitor. In some aspects of the methods, the anti-aging regimen comprises administration of an HIV inhibitor. In some aspects of the methods, the anti-aging regimen comprises administration of AZT. In some aspects of the methods, the anti-aging regimen comprises administration of an HBV inhibitor. In some aspects of the methods, the anti-aging regimen comprises administration of ribavirin. In some aspects of the methods, the anti-aging regimen comprises administration of a transposase inhibitor.

Some embodiments relate to methods for comparing a first nucleic acid sample and a second nucleic acid sample, comprising the steps of obtaining Mobile Element Insertion (MEI) border sequence for a plurality of MEI borders of the first nucleic acid sample, assaying for the presence of the plurality of MEI borders in the second nucleic acid sample, and identifying the second nucleic acid sample as different from the first nucleic acid sample if the second nucleic acid sample lacks an MEI border sequence present in the first nucleic acid sample. Some aspects of the methods comprise identifying the second nucleic acid sample as different from the first nucleic acid sample if the second nucleic acid sample includes an MEI border sequence not present in the first nucleic acid sample. In some aspects of the methods, obtaining Mobile Element Insertion (MEI) border sequence for a plurality of MEI borders of the first nucleic acid sample comprises performing whole-genome sequencing of the first nucleic acid sample. In some aspects of the methods, the obtaining Mobile Element Insertion (MEI) border sequence for a plurality of MEI borders of the first nucleic acid sample comprises performing targeted sequencing of the plurality of MEI borders of the first nucleic acid sample. In some aspects of the methods, assaying for the presence of the plurality of MEI borders in the second nucleic acid sample comprises performing whole-genome sequencing of the second nucleic acid sample. In some aspects of the methods, assaying for the presence of the plurality of MEI borders in the second nucleic acid sample comprises performing targeted sequencing of the plurality of MEI borders of the second nucleic acid sample. In some aspects of the methods, performing targeted sequencing of the plurality of MEI borders of the second nucleic acid sample comprises contacting the second nucleic acid sample with a panel of primers comprising primers that specifically amplify each MEI insertion site of the first nucleic acid sample. In some aspects of the methods, performing targeted sequencing of the plurality of MEI borders of the second nucleic acid sample comprises contacting the second nucleic acid sample with a panel of probes comprising probes that specifically anneal to each MEI insertion site of the first nucleic acid sample. In some aspects of the methods, the panel of probes comprises at least one probe bound to a fluorophore such that probe bound to substrate is differentially visualizeable relative to probe not bound to substrate. In some aspects of the methods, the second sample comprises a forensic sample. In some aspects of the methods, the second sample comprises a plant sample. In some aspects of the methods, the plant sample is a plant crop sample. In some aspects of the methods, the second sample comprises biohazardous substance.

Some embodiments relate to compositions for use in delaying age-related genome deterioration comprising a Mobile Element Insertion inhibiting pharmaceutical. In some aspects of the compositions, the composition comprises a reverse-transcriptase inhibitor. In some aspects of the compositions the composition comprises a retroviral inhibitor.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

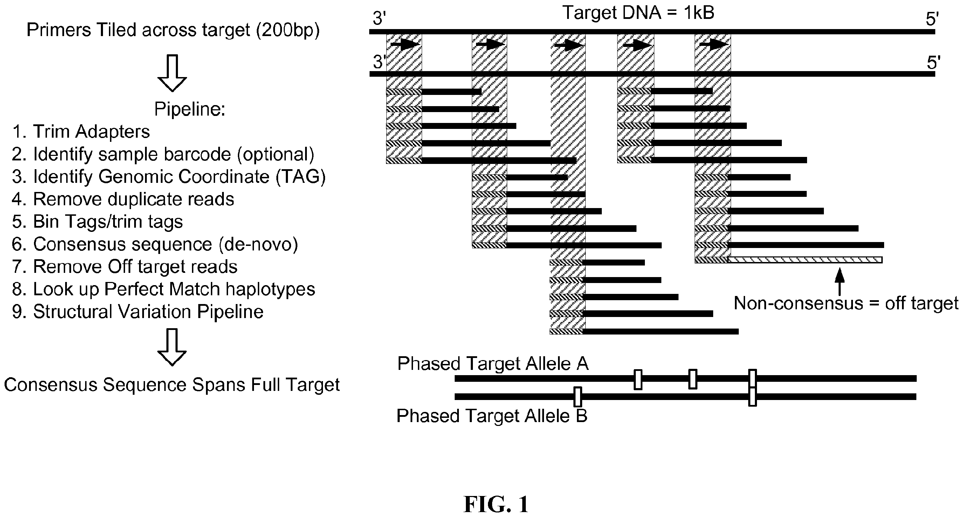

Figure 1 depicts the use of targeted sequencing to probe and/or detect complex variants.
Figure 2 represents the use of redundancy and labels to confirm and/or quantify insertion events.

### DETAILED DESCRIPTION

Mobile Element Insertions (MEI), also called transposable elements, make up two thirds of the human genome. There are hundreds of human genes that have evolved as a result of ancient MEI activity. Some MEIs are still active in the human genome, including modern Alu sequences. Neuronal cells have high MEI activity, and the effects of viral MEIs have a role in cancer on a genome wide scale. MEIs occur stochastically in both protein coding and non-coding regions of the genome without bias. They affect human host transcription and cellular activity and are therefore highly deleterious when disrupting the function of host genes. Strong negative selection occurs against germline transmission of these deleterious events. MEIs have been implicated in cancer and other genetic disorders, but the scale and scope of somatic MEIs have not been well studied or documented. New DNA sequencing technologies struggle to explain this effect because, for example, sample preparation and analysis methods are lacking the necessary sensitivity to quantify the effect of active MEIs in disease. Due to biased amplification, many such methods misrepresent the activity of MEIs. Methods for the accurate detection of MEIs need to be able to determine the critical genes that are affected by somatic MEIs and to quantify their activity as disease progresses. A non-invasive test to detect and quantify disruption of critical gene function is a universal test of cellular health and has implications in nearly all areas of adult onset disease.

Mobile DNA elements are a major driving force in evolution and genetic disease. Mobile elements comprise nearly two-thirds of the human genome. Major types of MEIs include but are not limited to Alu, LINE, SVA, type I retrotransposons, ERV (endogenous retrovirus) and, collectively, they are called the Mobilome.

Next generation sequencing technologies have increased our understanding of the prevalence of MEIs in the human genome. Alu, SINE and SVA elements are active in the human genome today. Specific families of MEIs have common sequence characteristics at their insertion sites, allowing for synthetic oligonucleotides to be produced to interrogate diagnostic sequences of these insertion events. Analysis of the inherited MEIs and their population frequencies within the publicly accessible data of the 1000 genomes project demonstrates that nearly all MEIs found in the study populations are considered rare and occurred in frequencies at less than 10%. Most inherited MEIs are not protein coding, indicating that MEIs are highly disruptive of gene function and are thus removed by natural selection. Particularly, somatic MEIs are tissue specific. For example, Alu and SVA MEIs are common tumor specific events, particularly in epithelial cancers, but unlikely to be found in blood or brain cancers. This indicates an environmental effect on MEI activity. Further evidence for the stress-induced activity of MEIs comes from the fact that many transposable elements have promoter sequences similar to heat shock TF binding sites. There is a correlation between the activation of MEs and a reduction in methylation, which has been proposed as a control mechanism for MEI activity. Somatic MEI activity is abundant in embryogenesis, tumor cell lines and neuronal progenitor cells, but little is known about the activity of MEIs in normal somatic tissue.

The HeLa cell line, a staple of cancer research for decades, has an HPV insertion site upstream of the c-Myc gene, which may be the cause of the indefinite cell division.

MEIs can alter human transcription by disrupting open reading frames or by providing alternative splice sites, alternative promoter sites or alternative polyA signals in human genes. With the draft of the human genome in 2001, many were surprised that the human genome only encoded for roughly 20,000 genes, especially when compared to the >100,000 translated proteins. Modern understanding implicates MEIs for this phenomenon, by introducing novel splice sites (e.g. L1, Alu). Most Alu derived genes are alternatively spliced and much of the alternative splicing is tissue specific. The majority of human genes utilize alternative splice sites, influenced by MEI processing of alternative ends of genes. For example, the ATRN gene has an L1 element in an intron. The alternatively spliced gene encodes a soluble form of Attractin, which is part of inflammation response. The alternative form acts as a receptor for pigmentation and energy metabolism. Over 120 retrotransposon sequences have evolved into functional human genes. The estimated rate of de novo germline MEI mutations ranges from 1 in 20 births to 1 in 100 to 1 in 1000 for Alu, L1 and SVA elements respectively. DNA methylation is shown to be a host defense mechanism and mice devoid of methyltransferase exhibit high chromosomal instability eventually becoming catastrophic. Small RNAs are also a regulatory mechanism of MEI activity and effect. These small RNAs, including classes of piRNA and siRNA are also MEI derived. The impact of MEI on transcription can be at a single locus through alternative splicing, alternative promoters or alternative polyA sites. On a global level, transcriptional networks are controlled by MEI promoter activity. Embryonic stem cells show the linking of gene networks. ES cells show a network of endogenous retroviral long tandem repeats that initiate a network of gene expression controlled by methylation. In a pluripotent state, the ERVs are repressed by methylation. Mammalian pregnancy pathways evolved through MEI activity. This gene network is activated by MER20 elements in progesterone response. For example, the prolactin promoter is derived from a MER39 mobile element. Synctin genes for fetal - maternal exchange are also derived from ERV genes. ERVs are flanked by LTRs of about 300 to about 1200 nucleotides. The size of many MEIs can range from, for example, about 200 bp to about 10kb for the more common elements.

ME-Scan can identify inherited MEIs from the AluYb8/9 elements, the most active common MEIs in the human genome. The method, however, is not quantitative in nature. These Alu elements mimic the diversity characteristics of SNPs when looking at African vs. European populations. Alu copies vastly outnumber coding genes in the human genome, and somatic MEI events were estimated to vastly outnumber germline events as the derepression of MEI activity occurs in tumors and senescent cells. The abundance and repetitive nature of these elements poses a problem to genome wide surveys at the time. The greater the sequencing depth, the greater the sensitivity to detect MEIs, but false positives may arise from the production of chimeric molecules during the library preparation step and certain elements may be overrepresented due to biases in processes like PCR.

While the above examples show the effect of inherited MEIs, their disruptive effect and negative selection, much has been learned about the prevalence of somatic MEIs. Mosaicism of MEIs is abundant in neuronal cells. Neurons have elevated levels of aneuploidy and retrotransposition, which may contribute to functional diversity in the human brain.

There is evidence to imply that the activation of MEIs in somatic tissue have an effect on aging. During normal aging, somatic MEI transcription starts to become active. The active retrotransposition of these elements occurs in advanced aging in mice, which corresponds to elevated genome instability as a function of cellular age. MEI location and abundance regulate the rate of aging, and the inability to maintain the complex DNA structures contributes to the dysfunction of tissues and the eventual demise of an organism. These transposition events can be accelerated by multiple stress-associated factors such as inflammation. Retrotransposition is in some cases mediated through inhibition of reverse transcriptase such as drugs used for hepatitis B virus (HBV) and human immunodeficiency virus (HIV) infections. Naturally occurring cancers in mice have an increase in MEI activity.

Cancer is and will most likely continue to be the most prevalent area of somatic MEI research as cancer is considered a disease of the genome. The effects of HBV integration events and their role in hepatocellular carcinoma (HCC) can be analyzed using high coverage whole genome sequencing (WGS). From a technical standpoint, an increased sequencing depth results in many more confirmed somatic insertion events with the number of insertional events proportional to the depth of sequencing. A clonal expansion in hepatocellular carcinoma (HCC) tumors results in higher frequency of the same events compared to normal samples from the same individuals. On average, in tumor derived DNA, two copies of the viral genome can be found for every one copy of the host human genome. Disruptive events are found near insertional sites, including direct gene disruption, viral promoter driven human gene transcription, viral -human transcript fusions, and DNA copy number alterations. There is support for a stochastic model of insertional events, suggesting that insertions are mostly random with perhaps the only influence being accessibility of DNA not bound up in chromatin. Previous PCR-based approaches often overestimate the prevalence of certain insertional events due to amplification biases. Integration is widespread throughout tumor and normal liver tissue, but there is a distinct pattern in tumors of insertional events in oncogenes and tumor suppressor genes where the functional impact is restricted to the tumor cells, and the abundance is a result of clonal expansion of these cells. The insertional "ends" map to distinct regions of the HBV genome, which can be used for detection purposes of the insertional site. For example, the DR1 and DR2 sites that are the direct repeat elements found at the end of the HBx gene in the HBV linear virus. The stochastic model shows that the fusion products from transcripts can map anywhere in the genome, so there is a common site from the virus and an unbiased site from the human genome at the insertion point.

Most HBV insertion sites are not recurrent, but there is a significant increase in abundance of Major Insertion Sites in the tumors that go through clonal expansion. Most events in the tumor occur near protein coding genes, suggesting that exposed DNA not in chromatin makes it accessible to insertion. There is a positive selection for insertion events in the tumor toward promoters and exons. Most insertions can appear to be neutral, not inserting into genes in the cancer gene census database. The number of integration sites in a tumor can correspond to outcome or other medical indicators such as survival. For example, tumors with >3 insertion events can have much greater negative effect on survival.

RNA-Seq data from tumors in various cancer types can be used to study host/viral fusions, especially known cancer causing viruses. For example, NGS RNA-Seq reads can be mapped to common viral strains of HPV, HBV, HCV, EBV and HHV to look at their effect on cervical cancer, liver cancer and Burkett's lymphoma. Using de novo assembly, negative cervical cancer tumors with novel HPV strains can be re-diagnosed. PCR assays may misrepresent the abundance of HPV integration. HPV positive integration can show tumor clustering regardless of tumor type or the tissue involved. Viral MEIs may cause cellular transformation by the expression of viral oncogenes or by integration to alter the activity of oncogenes or tumor suppressors.

For clinical utility of MEI detection and its diagnostic implications, a targeted strategy must be employed. MEIs are mosaic, caused by some form of environmental factor such as stress-induced activity of MEI or viral induced MEI activity. MEIs have been shown to have a dramatic impact on evolution as well as a highly deleterious effect when found in functional regions of the genome. The inheritance pattern models that of SNPs and the impact on somatic MEIs is a mystery just beginning to be unraveled. The ability to look at the insertional sites and quantify their abundance in all tissue types, perhaps using blood or cell free DNA in the blood as a surrogate, is a useful tool for determining cell health. The targets of these actively mobile or invading genomic elements can be used for both diagnostic purposes as well as rational therapeutic intervention for the specific individual through small RNAs, methylation or even inhibition of reverse transcriptase. Understanding the impact of these elements in an individual and specifically for an individuals' disease will enable new treatment and diagnostic options. For example, an MEI event causing cancer perturbs an oncogene or tumor suppressor. Fluorescent probes, activated through various means within the living tissue are used to target those cells for extraction in surgery. A probe spanning the junction of a mobile element and the human host gene sequence it perturbs, with binding efficiency only to the specific junction event, will provide a marker or beacon for extraction from surgery.

MEIs are likely non-human in origin, evolving over time or introduced by viral infection. Their main purpose, when looking from the perspective of a virus, is survival. Cellular stress induced by infection, inflammation, toxins such as alcohol, physical pressure, ulcers etc. all affect the survival of the cell and thus the activity of the MEIs increases. Deregulation of active MEIs could simply occur by chance. Active MEIs then rearrange chromosomes or alter cellular transcription. These effects can be modest or catastrophic. One cell gets derepressed and eventually expands clonally. The genes that are disrupted by the active MEI determine the rate of expansion. If cell growth genes or regulatory genes are disrupted, the rate of which they divide is increased, causing tumor growth. Conversely, non-cancer associated genes can also be perturbed and activate/deactivate critical cellular mechanisms (e.g. apoptosis, necrosis, proliferation, cell division). For example, if the apoptosis pathway is inactivated, the cells may continue to divide increase prevalence in the organ, and start to negatively impact the organs function. And eventually, this could lead to functional loss. The genes that are perturbed, as well as the number of insertional events could both act as diagnostic indicators of cell health or disease progression. In some cases, some of these cells may die, and the DNA from these cells will be found as cell free DNA in the blood. Monitoring the increase of these cell free molecules with a technology sensitive enough to detect these rare events, and accurate enough to quantify these rare events, will be a staple of molecular medicine. Both the number of events, starting with a baseline at an early age, as well as the genes they perturb, could catalog the cellular function of all human organs and monitor the cell health of an individual throughout their life. These diagnostics tests could lead to the early detection and prevention of nearly all adult onset diseases and disorders affected by MEI perturbations. The increased somatic activity in the brain could lead to cancer, neurodegenerative disorders such as Alzheimer's or Parkinson's, or other disorders such as autism. MEIs contain both an inherited component as well as somatic activity. The relationship between these could explain the missing heritability of many of these disorders and the stress or environmental induced activation of these elements. This may be an elegant explanation for these complex disorders.

MEIs represent the only truly individual genomic markers in our DNA. For twins carrying nearly identical DNA, the absolute difference cannot be detected using todays' sequencers due to the error rate. Conversely, searching for the MEI spectrum could determine the genomic differences that cause disease between two otherwise near identical genomes (e.g. twins). Further, the truly unique genetic makeup of MEIs constitutes the only truly unique forensic markers of identification. The simplest example is that of closely related individuals or twins. MEIs, in combination with traditional SNP testing or microsatellite markers, could definitely rule out even the closest genome sequences.

With the increasing amount of sequence information being generated on personal genomes, and an increasing willingness to share that information publicly, the ability to falsify genomic identity is certainly a reality. Synthetic DNA sequences with better binding affinity to primers for PCR analysis is in some cases generated from individuals sequence data accessed through the public domain, the research field, or even through a lack of cyber security at gene testing companies. Doping an individual's blood with these highly effective DNA sequences is unlikely to have an impact on the individual's health, or if it did, those may be willing to accept the risk. When blood is drawn for DNA testing, these more efficient molecules could confound or completely mask the identity of the individual or even represent the sample as another individual. MEIs, particularly those active recently as somatic, would present a completely unique identification strategy that is in some cases used as genomic identification both because of the position of these somatic events in the genome as well as the amount of such events in a complex background.

These forensic purposes could also be used in agriculture for detection of GMO crops. Seeds from a GMO farm could easily spread to neighboring farms. Many agriculture companies detect these transposed elements in neighboring farms to determine the extent of unwanted transfer of their intellectual property. Methods to quantify these diagnostic markers, with an extreme sensitivity, would allow the ability to detect and quantify the percent of organism that have been contaminated with their products. PCR methods and other biased strategies do not offer this level of sensitivity.

MEIs may have a major impact on the cosmetic industry as well. Since MEI activation is associated with cellular aging, it represents a unique way to study and determine the cause of wrinkles or hair loss. MEIs can be inherited, somatically activated or viral induced. All result in disruption of the genome and its function. Determining the genes that are perturbed represent new targets for therapeutics and cosmetic interventions to reduce or eliminate their activity. Monitoring the rate and level of MEI activity may be a signal for intervention, through natural means like calorie restriction, or through increased dosage of pharmacologic intervention.

A test to monitor cell health, starting with a baseline of MEI activity at an early age, as disclosed herein, is a frequent testing option for all individuals.

Throughout the specification herein, the disclosure is sorted into sections for ease of understanding. These divisions are understood to be for ease of understanding and not necessarily to limit the applicability of some sections of the specification with respect to one another. Accordingly, disclosure in any one section of the specification is relevant in some cases not only to that section but to other sections and in some cases to the disclosure as a whole.

### Methods for somatic MEI detection and quantification

Current whole genome methods for MEI detection involve whole genome sequencing and bioinformatics analysis. MEI events cause "split-reads" where a portion of the sequence maps to the human reference genome and the other portion does not map properly. Mate pairs or paired end reads offer the ability to use all or a portion of one read to anchor the position of the unmapped or linked portion of the DNA molecule. Massively parallel sequencing allows for redundant interrogation and an increased level of confidence through greater sampling. However, that increased sampling comes at a dramatically increased cost. Deeper sequencing depth is proportional to the sensitivity for MEI detection. Whole genome sequencing (WGS) approaches pose a problem with increased cost as well as unwanted data and ethical considerations, but have the advantage of unbiased detection of MEI insertion sites throughout a sample in some cases. In some cases, these methods introduce sequence specific amplification biases that would inhibit the ability to quantify some MEI events, which is critical to determine the difference between a neutral MEI and a disease causing MEI.

Some previous targeted methods for MEI generally involve a variant of hemi-specific PCR. These methods, as previously discussed, are not quantitative in some cases due to sequence specific biases, dramatically over representing the quantity of some MEI locations over others due to sequence amplification efficiency. There is no way to determine if a somatic MEI event is neutral and therefore represented stochastically (randomly) or if it has been clonally expanded such as in cancer. In addition, there is limited flexibility in the design of a locus specific primer for the insertional end of the MEI. If the sequence is mutated or differs by enough to cause no amplification or less efficient amplification, then quantifying the amount of that specific event is not possible. Thus care must be taken when using these methods to ensure that sequence results quantitatively reflect the amounts of templates in the original nucleic acid sample.

Some appropriate targeted somatic MEI detection methods are able to provide redundancy as the insertional ends of MEIs are often repetitive or altered. Active somatic MEIs are modern and less likely to be truncated compared to ancient inactive MEIs, but they could be mutated or minimized in terms of the diagnostic sequences. Therefore, multiple redundant locus specific primers are designed against the insertional ends of MEIs, such as TSRs or the DR 1 / 2 diagnostic regions near the Hbx gene in the case of HBV. These multiple different starting points also allow for the confirmation of MEIs as multiple independent samplings of an MEI event allow for an internal confirmation of the event and greater sensitivity and specificity. In addition, natural labels, or alternative 3' ends should be produced for NGS library molecules. The combination of redundant primer sites and the natural labels due to alternative 3' ends of NGS library molecules shows independent sampling of the DNA templates, insuring that any localized insertion events can be confirmed and quantified through the removal of clonal artifacts during the amplification steps. In addition, such methods need to avoid fragmentation and ligation in the preparation process as chimeric molecules could be produced during these preparation steps and result in false positives.

Other quantification methods are contemplated herein, and the methods disclosed herein relating to MEI sites are not limited by any single quantification method. Various methods are presented herein as alternatives, highlighting challenges and advantages that each presents, and precautions to be taken to make each approach applicable to the methods disclosed herein.

Various embodiments of the disclosure herein involve quantification of one or more MEI events in relation to their insertion-adjacent genomic sequence. Quantification is accomplished by a number of approaches. MEIs, sometimes referred to MEIs and their insertion-adjacent genomic sequences, are initially identified by whole genome sequencing in an untargeted approach, or by specific or hemi-specific PCR or other approaches known in the art. TAIL-PCR or other approaches known in the art for determining insert-adjacent sequence are used in some embodiments. In many embodiments, whole genome sequencing or other untargeted approaches are preferred for initial MEI mapping to insertion adjacent borders. In follow up assays, whole genome approaches are used in some embodiments, while targeted assays for specific MEI and insertion adjacent sequence are used in alternate follow-up assays or in combination with whole genome assays.

Quantification of the abundance of a MEI-insert -adjacent sequence junction in a nucleic acid sample is effected by a number of alternate or coordinate approaches. Specific MEI insert borders are quantified by comparing the number of reads, or the number of unique reads, or the number of independently derived reads, spanning a given MEI and its insert-adjacent sequence to any one or more of the following: the amount of nucleic acid in the sample; the number of reads, or the number of unique reads, or the number of independently derived reads mapping to a known single-copy sequence in the nucleic acid sample, the number of reads, or the number of unique reads, or the number of independently derived reads mapping to a separate MEI and its insertion adjacent sequence; or the number of reads, or the number of unique reads, or the number of independently derived reads mapping to the same MEI and its insert adjacent sequence at a different time point. In some cases, a specific MEI insertion site is quantified by measuring the number of independent reads spanning its insertion site relative to the total amount of input nucleic acid. In some cases, a specific MEI insertion site is quantified by measuring the number of independent reads spanning its insertion site relative to the number of independent reads mapping to a known unique locus of the nucleic acid sample. In some cases, a specific MEI insertion site is quantified by measuring the number of independent reads spanning its insertion site relative to the number of independent reads mapping to a multicopy locus of known copy number. In some cases, a specific MEI insertion site is quantified by measuring the number of independent reads spanning its insertion site from a sample at a first time point relative to the number of independent reads spanning its insertion site from a sample at a second time point. Alternate quantification methods, such as quantification by hybridization to fluorescent probes having quantifiable fluorescence levels, are contemplated in combination or as alternatives.

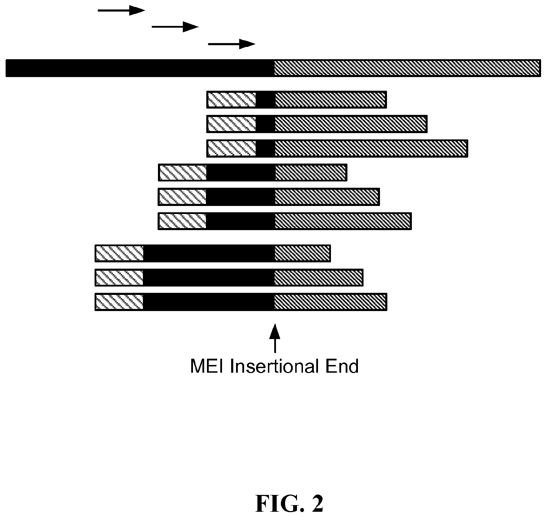

Figure 2 presents an example of multiple independent reads for use in quantification of a MEI insertion site. Each read comprises MEI and insertion adjacent sequence, and each read has a unique combination of 5' end, 3' end and insertion length. Thus, each read can be identified and an independent representation of the MEI and insertion adjacent sequence rather than a clonally amplified PCR product.

### Design

Each family of MEI has similar sequences at the insertional ends of the MEI. For example, in Alus, there can be a 7bp diagnostic sequence flanking a repeat sequence. The length of the ends can be variable and/or can have some repetitive sequences. In Alu sequences, there can also be stretches of polyA sequences. The polyA sequences can partially be targeted. Direct repeat regions that have sequence homology, such as DR1 and DR2, can also be targeted. Using longer read length (e.g. MiSeq 2x350 reads), longer inserts for paired end sequencing (e.g. 500 bp inserts) and a controllable fragment length due to ddNTP incorporation, multiple primers can be designed to each strand of the DR1 and DR2 regions of mobile elements. For example, to target a 1kb region at each end of the mobile elements (e.g. Alus, LINEs, SVA, viral MEIs, etc), multiple non overlapping primers can be designed to span from the very end (near the terminal repeats) through more complex sequences to provide greater specificity. In some cases, at least about three primers can be used for each flanking element of the MEIs. Unlike PCR, due to the linear primer extension with a strand displacing polymerase, the multiple priming sites will not interfere with each other. Each family of elements may have enough sequence disparity to immediately identify the element type by sequencing through the synthetic sequences generated. The multiple primers within each element family can be identified and binned together for self-assembly. In some cases, the reads can be mapped with enough certainty to determine if there is an interruption to a critical gene. Multiple primers for the same MEI then can be used as independent confirmation of the same MEI disruption event by simply comparing the non-MEI sequence produced from the chimeric molecule. Along with multiple priming events (e.g. about 3 to about 10 per MEI), each single primer will produce multiple copies of the same event from multiple copies of the genome. The natural labels and the 3' synthetic labels can be used to determine the independent samplings of the template, and further confirm the event. Interestingly, the same method can be used to determine the relative age of the event. More ancient MEI events tend to have truncated ends or mutations within the MEI sequence itself, and these events are typically shown as being inactive because they lack the insertional sequences needed for cut and paste or copy and paste activity.

The present disclosure also provides methods for detecting MEIs in inherited diseases. In the presence of a full length MEI, other sources of data can be used to further determine whether the MEI is somatic or still active. In general, a truncation event can indicate an inactive MEI, which is likely to be inherited and found in majority of molecules. On the other hand, novel somatic MEI activity (which can be an indication of cellular aging) is found in a smaller percent of molecules, which is the reason extreme sequence depth is needed. Via back calculations, the rate of somatic activity is approximately about one in 25 cell divisions. In a heterozygous population, this translates to about one in 50 DNA molecules from a given tissue or biopsy. For a one in 50 event, with three reads for each individual event, a sequencing depth of 150 x is required. In view of potentially high heterogeneity and the fact that many of these are singleton events, it is possible that at least 1000 fold coverage on average may be provided to analyze tumors, and perhaps even higher than that for analyzing endogenous activity for ancient MEIs such as Alus (e.g. about 1 million fold coverage).

The other sources of the data are from the gene or genes they perturb, but also from the number of events and if the events are clonally amplified. For example, viruses such as HPV or HBV will randomly insert themselves to many regions of the genome. It is a stochastic event that leads to an even level of normalized coverage across each individual event. If the event hits a cell growth gene (e.g. oncogene or tumor suppressor), then clonal expansion of those cell types may be observed. So the number of specific MEI events, when compared to the number of background singleton or doubleton somatic events, acts as an indicator of disease diagnosis. For single cell work, multiple events in the same cell can be an indicator of outcome as it has been shown in tumors. Even looking at a heterogeneous tumor may provide another level of data as each tumor may have a collection of infected but non-tumor cells. The ratio of background events to tumor-causing events can be calculated by averaging the sequencing depth coverage across each of the events. An increase of 3 fold or greater, for example, would be a cutoff of a tumor-causing vs benign event. This can be used as a monitoring target for the specific tumor in the blood during treatment, or to determine disease progression, or to be used as a probe (spanning the event) for extraction during surgery to insure removal of the tumor is complete. For example, in a case of HBV infection in liver cells, 3 different primers targeting the insertional end of the DR1 or DR2 regions near the Hbx gene in the linear virus can be used to identify all of the reads with the specific primers for the HBV sequence. By calculating the average coverage depth from each of the three primers that produced data from a given location in the genome, and comparing the average depth of a given event to the average depth of the other random insertional events, the higher average coverage event can be highlighted as a major insertion site that may lead to clonal expansion. In some examples, the average depth of the given event can be more than about 1.2 times, about 1.4 times, about 1.6 times, about 1.8 times, about 2 times, about 3 times, about 4 times, about 5 times, about 10 times, about 20 times, about 50 times, or about 100 times the average depth of the other random insertional events. The three different primers can be used to remove amplification artifacts from more efficient sequences (e.g. lower GC content regions). The natural and random synthetic labels can be used to remove clonal amplification of any one event. In sum, there may be multiple sources of information to confirm and quantify each event.

The present disclosure can provide a composition comprising a library of molecules each representing MEI events. The library can be in a multiplex format.

The present disclosure can further provide a method to test for all known cancer causing viruses and/or all known active ALUs and MEIs that are passed through to generations in the germline. The method can be used for applications such as cancer gene disruption, cellular aging, disruption of critical genes in each tissue specific MEI event (e.g. Alzheimer's in aging brains), and/or testing for cellular health and aging.

The present disclosure provides a method to generate an unknown sequence in the genome from a known insertional site sequence. The unknown sequence can be used to determine the disruption of a gene. The synthetic primer sequences from the read can be used to determine the MEI type sequenced, the genomic sequence can be used to identify the disrupted gene, and the natural and synthetic labels can be used to determine the quantitative amounts of each event. Therefore, position and abundance of the event as well as the overall activity (total number of events) can all have diagnostic or prognostic implications in adult onset disease and cellular health.

Primer design for regions of insertion occurs in known diagnostic sequences at the 5' or 3' insertional sites of MEIs. Broken up into windows of 20, 50, 100 base pairs, a unique or somewhat unique primer sequence is designed taking into account TM, degenerate positions and repetitive positions. Primer design is redundant in that multiple primers starting from the insertional end are designed. A single primer library designed against all known MEI viral and endogenous MEI sequences is developed, synthesized and pooled in equimolar ratios.

Primers include a molecular "tail" at the 5' end corresponding to an adapter compliment of the sequencing platform being used. An optional molecular barcode is included in the synthesis step for sample multiplex in some cases.

Primer extension occurs through the use of a strand displacing polymerase at uniform temperature or through the use of a thermal stable polymerase and cycling of the primer extension reaction. The polymerase must have the ability to extend while incorporating modified bases or bases with a terminal 3' end lacking a hydroxyl group.

A combination of native dNTPs and biotinylated ddNTPs are used in the reaction mix. The ratio of ddNTP to native dNTP determines the fragment length of the extended molecule. For example, using 1% fraction of ddNTP would produce a 1/100 chance of incorporating a terminating molecule at any given base. Typical results show that a 1% ddNTP ratio produces fragment peaks around 500bp. This is likely due to the efficiency differences in incorporation of native vs. altered NTPs.

The resulting molecule is a chimeric consisting of synthetic sequence at the 5' end and patient derived sequence at the 3' end. The molecule ends with a terminated, biotinylated nucleotide.

The molecules are purified from the genomic background through the use of an affinity reaction. Streptavidin coated magnetic beads are used for this step. Four biotinylated molecules bind per streptavidin molecule on the bead and the remaining ddNTPs, dNTPs and unused primers are removed.

A second primer extension reaction occurs through the use of a random primer consisting of 8 nucleotides at the 3' end and the B-adapter compliment corresponding to the sequencer platform. Random priming occurs across the molecule but through the use of a strand displacing polymerase, only the most distal random primer and its extended product will remain hydrogen bound to the streptavidin bead. The copied molecule from the B reaction will run all the way through the A primer on the previous strand and produce a single stranded molecule with a 5' B adapter, 8 bp synthetic random sequence, human host genome sequence site of MEI insertion, synthetic sequence of MEI locus specific primer and the A adapter compliment at the 3 end. These molecules are denatured from the streptavidin bound molecule and PCR amplified to incorporate the full-length sequencer adapters and an optional external bar code if sample multiplex is required.

This chimeric read structure and its features have many advantages in data analysis. The synthetic locus specific sequence of the primer is used to determine which MEI is targeted in the read. The redundant primer sites resulting in different extension start points for the same MEI species can be used as an internal confirmation of insertional events. This also avoids drop out for less efficient or inappropriately designed locus specific primers. The locus specific primers can be used for all known MEIs including Alus, LINEs as well as viral MEIs. The full spectrum of known viruses would be designed in a single library with the likelihood of multiple viruses in the same sample being low. It is likely that many of the viral primers will not produce data in any given sample.

The 3' fragmentation and altered 3' sequence acts as an internal molecular label or a natural barcode. If two reads have different natural labels (3' sequences) then they are certain to be independent reads off of the template DNA and NOT clonal errors.

The random 8 bp of synthetic sequence from the B adapter reaction can also act as a stochastic label. The combination of the random 3' sequence and the stochastic label from the random 8-mer can be used in combination to further insure that the reads are independent and not clonally amplified.

During data analysis, the reads from a given MEI are first trimmed of adapter sequences. Molecular barcodes are identified if the sequencer run contained multiplexed barcoded samples. The first 5-25 bases corresponding to the synthetic locus specific primer are identified to determine the MEI event being targeted. The bases are then trimmed from the read for mapping and assembly. The remaining sequences are mapped against the human reference genome and assembled across overlapping reads to provide the evidence for the insertional location in the human genome. Duplicate reads are removed based on their 3' ends and the stochastic labels. For paired end reads, the 2nd read is recruited if not individually mapped with the insert size being reduced to provide overlapping reads against the insertional site. Using the MiSeq system, with 300 bp insert sizes (preferential to the ILMN cluster generation) a cumulative sequence of about 400-500 bp is generated for positional mapping. After all clonal reads are removed, the position and number of events are quantified for each position.

Accordingly, disclosed herein are methods, compositions and methods of use related to Mobile Element Insertion (MEI) insertion site sequences and mobile element activity, for example as they relate to human health. Human mobile elements can be categorized as DNA transposons or retrotransposons. DNA transposons move by a cut-and-paste mechanism. Retrotransposons mobilize by a copy-and-paste mechanism via an RNA intermediate, a process called retrotransposition.

Mobile elements implicated in human disease are known in the art. Exemplary mobile elements include, without limitation, L1, Alu, SINE-R/VNTR/Alu (SVA), processed pseudogenes, and human endogenous retrovirus (HERV). Retrotransposons located 5' of protein coding loci frequently function as alternative promoters. For example, retrotransposons located in the 3' UTR (untranslated region) of genes show strong evidence of reducing the expression of the respective gene, as assessed by cap analysis gene expression and pyrosequencing. Hypomethylation of retrotransposons is known to affect either the transcription of the retrotransposon itself or that of nearby genes. For example, increased methylation of a promoter in L1 associated with the *MET* (hepatocyte growth factor receptor) oncogene is known to induce an alternative MET transcript within the urothelium of tumor-bearing bladders.

Similarly, a number of human retroviruses constitute 'mobile elements' are contemplated herein due to their impact on human genomic sequence. A number of human retroviruses are known in the art. Retroviruses are known to exist in two forms: as normal genetic elements in their chromosomal DNA (endogenous retroviruses) and as horizontally-transmitted infectious RNA-containing viruses which are transmitted from human-to-human (exogenous retroviruses, e.g. HIV and human T cell leukemia virus, HTLV). Aberrant changes to DNA due to human retrovirus insertion are known to be associated with the onset of disease. Exemplary human retroviruses that insert into human DNA include, without limitation, HIV1, HIV2, HTLV1, HTLV2, and HSRV.

Disclosed herein are methods of identifying mobile element insertion (MEI) tagged cell proliferation. In some cases these methods comprise the steps of quantitatively measuring MEI levels at a first MEI insertion site in a first nucleic acid sample; quantitatively measuring MEI levels at a first MEI insertion site in a second nucleic acid sample; and identifying the first MEI insertion site as tagging MEI tagged cell proliferation if MEI levels at a first MEI insertion site in a first nucleic acid sample differ substantially from MEI levels at a first MEI insertion site in a second nucleic acid sample.

In some cases sample nucleic acid amounts are normalized, while in alternate cases nucleic acid amounts are normalized by, for example, measuring levels of one or a plurality of nucleic acids known in healthy individuals to be present at a single copy per haploid genome. In some cases, 'differing substantially' occurs when to samples differ in nucleic acid abundance or nucleic acid relative abundance or normalized nucleic acid abundance by 5%, 10%, 15%, 20%,25%, 30%, 35%, 40%, 45%, 50%, or greater than 50%. In some cases, 'differing substantially' refers to differing by 5%. In some cases, 'differing substantially' refers to differing by 10%. In some cases, 'differing substantially' refers to differing by 15%. In some cases, 'differing substantially' refers to differing by 20%. In some cases, 'differing substantially' refers to differing by 25%. In some cases, 'differing substantially' refers to differing by 30%. In some cases, 'differing substantially' refers to differing by 35%. In some cases, 'differing substantially' refers to differing by 40%. In some cases, 'differing substantially' refers to differing by 45%. In some cases, 'differing substantially' refers to differing by 50%. In some cases, 'differing substantially' refers to differing by greater than 50%.

Sequence adjacent to an MEI insertion site is determined in some cases. Sequence adjacent to an MEI insertion site is used in some cases to select a treatment, for example if that MEI insertion is associated with hyper-proliferation relative to other MEIs or at one point over a previous time point.

For example, if the MEI-adjacent sequence corresponds to a known oncogene, then a treatment associated with addressing cancers associated with that oncogene are selected to be administered to an individual demonstrating a hyper-proliferation either temporally or spatially of that MEI.

A number of genes associated with the onset of cancer are known in the art. These genes are known by various names, including, without limitation, cancer drivers, oncogenes, tumor suppressors and tumor susceptibility genes. Aberrant DNA changes in these genes are known to contribute to cancer progression. Exemplary genes that when altered are associated with driving cancer include, without limitation, abl1, acvrlb, af4/hrx, akt1, akt-2, alk, alk/npm, aml1, aml1/mtg8, apc, ar, aridla, aridlb, arid2, asxl1, atm, atrx, axin1, axl, b2m, bap1, bcl2, blc-3, bcl-6, bcor, bcr/abl, braf, brca1, brca2, card11, casp8, c-myc, cbl, cdc73, cdh1, cdkn2a, cebpa, cic, crebbp, crlf2, csflr, ctnnb1, cyld, daxx, dbl, del/can, dnmt1, dnmt3a, e2a/pbx1, egfr, enl/hrx, ep300, erbB, erbB-2, erg/TLS, ets-1, ews/fli-, ezh2, fam123b, fbxw7, fgfr2, fgfr3, flt3, fms, fos, foxl2, fps, fubp1, gata1, gata2, gata3, gli, gna11, gnaq, gnas, gsp, her2/neu, h3f3a, histlh3b, hnfla, hras, hox11, hst, idh1, idh2, il-2, int-2, jak1, jak2, jak3, jun, kit, ks3, K-sam, kdm5c, kdm6a, kit, klf4, kras, lbc, lck, lmo1, lmo2, l-myc, lyl-1, lyt-10C alpha-1, mas, mdm-2, mos, map2k1, map3k1, med12, men1, met, mlh1, mll2, mll3, mpl, msh2, msh6, myd88, myb, myh11/cbfb, ncor1, neu, n-myc, nf1, nf2, nfe2l2, notch1, notch2, npm1, nras, ost, pax5, pbx1/e2a, pbrm1, pdgfra, phf6, pik3ca, pik3r1, pim-1, prad-1, ppp2rla, prdm1, ptch1, pten, ptpn11, raf, rar/pml, rasH, rasN, rb1, rel/nrg, ret, rhom1, rehom2, ros, rnf43, runx1, ski, sis, set/can, srcret, setd2, setbp1, sf3b1, smad2, smad4, smarca4, smarcb1, smo, socs1, sox9, spop, srsf2, stag2, stk11, tall, tal2, tan-1, tiam1, tsc2, trk, tet2, tnfaip3, traf7, tp53, tsc1, tshr, u2af1, vhl, and wt1. An MEI insertion adjacent sequence that maps to a gene in this list, for example, suggests in some cases that a treatment associated with said gene or with a signaling pathway in which said gene's gene product participates is to be selected for incorporation into a treatment regimen.

Similarly, a number of genomic rearrangements are identified as being involved in cancer. It is known in the art that gene rearrangements in cancer arise primarily from DNA double-strand breaks (DSBs). Exemplary mechanisms leading to gene rearrangement include, without limitation, synthesis-dependent end-joining (SDEJ), sister chromatid fusion caused gene amplification by breakage-fusion-bridge cycles, V(D)J recombination-activating (RAG) proteins mediated translocation, and activation-induced cytidine deaminase (AID) class switch recombination.

Exemplary gene rearrangements include, without limitation, ACSL3/ETV1, ACTB/GLI1, AFF3/BCL2, AGTRAP/BRAF, AHRR/NCOA2, AKAP9/BRAF, ALK/PTPN3, ANKRD28/NUP98, ARHGAP6/PRCC, ASPSCR1/TFE3, ATIC/ALK, BACH2/BCL2L1, BCL11B/TCR, BCL2/Ig, BCOR/RARA, BCR/ABL1, BCR/FGFR1, BCR/JAK2, BCR/PDGFRA, BIRC3/MALT1, BRD3/C15orf55, BRWD3/ARHGAP2, BRWD3/ARHGAP20, C11orf95/MKL2, C15orf21/ETV1, C15orf55/BRD4, C6orf204/PDGFRB, CACNA2D4/WDR43, CANT1/ETV4, CAPRIN1/PDGFRB, CARS/ALK, CBFB/MYH11, CCDC6/PDGFRB, CCDC6/RET, CCDC88C/PDGFRB, CCND1/FSTL3, CD44/SLC1A2, CD74/ROS1, CDH11/USP6, CDK5RAP2/PDGFRA, CDK6/MLL, CEP110/FGFR1, CHCHD7/PLAG1, CHIC2/ETV6, CIC/DUX4, CLTC/ALK, CLTC/TFE3, CNBP/USP6, CNTRL/KIT, COL1A1/PDGFB, COL1A1/USP6, COL1A2/PLAG1, COL6A3/CSF1, CREB3L2/PPARG, CRTC1/MAML2, DGKB/MIPOL1, EML1/ABL1, EML4/ALK, EPC1/PHF1, ERC1/PDGFRB, ESRP1/RAF1, ETV6/ABL1, ETV6/ABL2, ETV6/ACSL6, ETV6/ARNT, ETV6/BAZ2A, ETV6/CDX2, ETV6/FGFR3, ETV6/FLT3, ETV6/GOT1, ETV6/ITPR2, ETV6/JAK2, ETV6/LYN, ETV6/MDS2, ETV6/MECOM, ETV6/NKAIN2, ETV6/NTRK3, ETV6/PDGFRA, ETV6/PDGFRB, ETV6/PER1, ETV6/PRDM16, ETV6/RUNX1, ETV6/SYK,EWSR1/ATF1, EWSR1/CREB1, EWSR1/DDIT3, EWSR1/ERG, EWSR1/ETV1, EWSR1/ETV4, EWSR1/FEV, EWSR1/FLI1, EWSR1/NFATC2, EWSR1/NR4A3, EWSR1/PATZ1, EWSR1/PBX1, EWSR1/POU5F1, EWSR1/SMARCA5, EWSR1/SP3, EWSR1/WT1, EWSR1/ZNF444, EXOC2/IGH, FCHSD1/BRAF, FGFR1OP/FGFR1, FGFR1OP/FGFR1, FGFR1OP2/FGFR1, FIP1L1/PDGFRA, FIP1L1/RARA, FOXO1/PAX3, FOXP1/ABL1, FUS/ATF1, FUS/CREB3L1, FUS/CREB3L2, FUS/DDIT3, FUS/ERG, FUS/FEV, FZD6/SDC2, GAPDH/BCL6, GIT2/PDGFRB, GOLGA4/PDGFRB, GOLGA5/RET, GOPC/ROS1, HAS2/PLAG1, HELIOS/BCL11B, ERVK-17/ETV1, HIP1/PDGFRB, HIST1H4I/BCL6, HMGA1/LAMA4, HMGA2/CCNB1IP1, HMGA2/COG5, HMGA2/COX6C, HMGA2/FHIT, HMGA2/ LPP, HMGA2/NFIB, HMGA2/RAD51L1, HMGA2/WIF1, HMGN2P46/ETV1, HNRNPA2B1/ETV1, HOOK3/RET, HPR/MRPS10, HSP90AA1/BCL6, HSP90AB1/BCL6, IKZF1/BCL6, IL2/DEXI, IL2/TNFRSF17, IL21R/BCL6, INPP5D/ABL1, ITK/SYK, Ig/BCLllB, Ig/BCL3, Ig/BCL6, Ig/BCL7A, Ig/CCND1, Ig/CCND3, Ig/CDKN2A, Ig/FCGR2B, Ig/FCRL4, Ig/FOXP1, Ig/IL3, Ig/KDSR, Ig/LHX4, Ig/LHX4, Ig/MUC1, Ig/MYC, Ig/PAFAH1B2, Ig/WHSC1, Ig/WWOX, JAZF1/PHF1, JAZF1/SUZ12, KIAA1549/BRAF, KIF5B/ALK, KIF5B/PDGFRA, KIF5B/RET, KLK2/ETV4, KTN1/RET, LCK/TCR, LCP1/BCL6, LE01/SLC12A1, LIFR/PLAG1, LRRFIP1/FGFR1, LYL1/TCR, MALAT1/ACAT2, MALAT1/TFEB, MALT1/MAP4, MEF2D/DAZAP1, MIR142/MYC, MLL/ABI1, MLL/ABI2, MLL/ACACA, MLL/AFF1, MLL/AFF3, MLL/AFF4, MLL/ARHGAP26, MLL/ARHGEF12, MLL/CASC5, MLL/CASP8AP2, MLL/CBL, MLL/CREBBP, MLL/DAB2IP, MLL/EEFSEC, MLL/ELL, MLL/EP300, MLL/EPS15, MLL/FLNA, MLL/FOXO3, MLL/GAS7, MLL/GMPS, MLL/GPHN, MLL/KIAA0284, MLL/KIAA1524, MLL/LASP1, MLL/LPP, MLL/MAML2, MLL/MAPRE1, MLL/MLLT1, MLL/MLLT10, MLL/MLLT11, MLL/MLLT3, MLL/MLLT4, MLL/MLLT6, MLL/MYOIF, MLL/NCKIPSD, MLL/NEBL, MLL/PICALM, MLL/PDS5A, MLL/SACM1L, MLL/SEPT11, MLL/SEPT2, MLL/SEPT5, MLL/SEPT6, MLL/SEPT9, MLL/SH3GL1, MLL/SORBS2, MLL/TET1, MLL/ZFYVE19, MN1/ETV6, MSI2/HOXA9, MSN/ALK, MYB/GATA1, MYB/NFIB, MYC/Ig, MYC/ZBTB5, MYH9/ALK, MYO18A/FGFR1, MYST3/ASXL2, MYST3/CREBBP, MYST3/NCOA2, MYST3/NCOA3, MYST4/CREBBP, NAV2/TCF7L1, NCOA4/RET, NDE1/PDGFRB, NDRG1/ERG, NDRG1/ERG, NFKB2/INA, NFKB2/TBXAS1, NIN/PDGFRB, NONO/TFE3, NOTCH1/TCR, NPM1/ALK, NPM1/MLF1, NPM1/RARA, NSD1/ANKRD28, NUMA1/RARA, NUP214/ABL1, NUP214/DEK, NUP98/ADD3, NUP98/CCDC28A, NUP98/DDX10, NUP98/HHEX, NUP98/HMGB3, NUP98/HOXA11, NUP98/HOXA13, NUP98/HOXA9, NUP98/HOXC11, NUP98/HOXC13, NUP98/HOXD11, NUP98/HOXD13, NUP98/IQCG, NUP98/KDM5A, NUP98/LNP1, NUP98/MLL, NUP98/NSD1, NUP98/PRRX1, NUP98/PRRX2, NUP98/PSIP1, NUP98/RAP1GDS1, NUP98/SETBP1, NUP98/TOP1, NUP98/WHSC1L1, OMD/USP6, P2RY8/CRLF2, PAX3/NCOA1, PAX3/NCOA2, PAX5/AUTS2, PAX5/BRD1, PAX5/C20orf112, PAX5/DACH1, PAX5/ELN, PAX5/ETV6, PAX5/FOXP1, PAX5/HIPK1, PAX5/JAK2, PAX5/PML, PAX5/POM121, PAX5/SLCOIB3, PAX5/ZNF521, PAX8/PPARG, PCM1/JAK2, PCM1/RET, PDE4DIP/PDGFRB, PEX5/LPL, PICALM/MLLT10, PIM1/BCL6, PML/RARA, POU2AF1/BCL6, PPP2R2A/CHEK2, PRKAR1A/RARA, PRKAR1A/RET, PRKG2/PDGFRB, PVRL2/TCR, RABEP1/PDGFRB, RANBP17/TCR, RANBP2/ALK, RBM15/MKL1, RBM6/CSF1R, RCSD1/ABL1, RNF213/ALK, RPN1/MECOM, RUNX1/AFF3, RUNX1/CBFA2T3, RUNX1/CLCA2, RUNX1/LPXN, RUNX1/MACROD1, RUNX1/RUNX1T1, RUNX1/SH3D19, RUNX1/TRPS1, RUNX1/USP42, RUNX1/YTHDF2, RUNX1/ZNF687, RYK/ATP5O, SEC31A/ALK, SEC31A/JAK2, SENP6/NKAIN2, SET/NUP214, SFPQ/ABL1, SFPQ/TFE3, SFRS3/BCL6, SLC34A2/ROS1, SLC45A3/BRAF, SLC45A3/ELK4, SLC45A3/ERG, SLC45A3/ETV1, SLC45A3/FLI1, SNX2/ABL1, SPECC1/PDGFRB, SPTBN1/FLT3, SQSTM1/ALK, SRGAP3/RAF1, SS18/SSX1, SS18/SSX2, SS18/SSX4, SS18L1/SSX1, SSBP2/JAK2, STAT5B/RARA, STRN/PDGFRA, TAF15/NR4A3, TAF15/ZNF384, TAL1/RHOA, TAL1/TCR, TCEA1/PLAG1, TCF12/NR4A3, TCF3/HLF, TCF3/NOP2, TCF3/PBX1, TCF3/TFPT, TCF3/ZNF384, TCR/LMO1, TCR/LMO2, TCR/MTCP1NB, TFG/ALK, TFG/NR4A3, TFG/NTRK1, TFRC/BCL6, THRAP3/USP6, TLX1/TCR, TMPRSS2/ERG, TMPRSS2/ERG, TMPRSS2/ETV1, TMPRSS2/ETV4, TMPRSS2/ETV5, TP53BP1/PDGFRB, TPM3/PDGFRB, TPM4/ALK, TPR/NTRK1, TRIM24/FGFR1, TRIM27/RET, TRIM33/RET, TRIP11/PDGFRB, VTI1A/TCF7L2, WDR48/PDGFRB, WWTR1/CAMTA1, ZBTB16/RARA, ZMIZ1/ABL1, ZMYM2/FGFR1, RUNX1/KIAA1549L, YAP1/TFE3, GTF2I/NCOA2, EWS/FLI1, SLC44A1/PRKCA, NAB2/STAT6, CUX1/AGR3, FGFR3/BAIAP2L1, FGFR3/TACC3, FGFR3/TACC3, and NABPl/RARA. Thus an MEI insertion-adjacent sequence that corresponds to a gene implicated in an oncogenic rearrangement is suggestive that a treatment associated with the rearrangement will be efficacious in a treatment regimen for the individual.

In some cases, an anticancer agent is administered based on information obtained from genomic analysis. Examples of chemotherapeutic anticancer agents include Nitrogen Mustards like bendamustine, chlorambucil, chlormethine, cyclophosphamide, ifosfamide, melphalan, prednimustine, trofosfamide; Alkyl Sulfonates like busulfan, mannosulfan, treosulfan; Ethylene Imines like carboquone, thiotepa, triaziquone; Nitrosoureas like carmustine, fotemustine, lomustine, nimustine, ranimustine, semustine, streptozocin; Epoxides like etoglucid; Other Alkylating Agents like dacarbazine, mitobronitol, pipobroman, temozolomide; Folic Acid Analogues like methotrexate, permetrexed, pralatrexate, raltitrexed; Purine Analogs like cladribine, clofarabine, fludarabine, mercaptopurine, nelarabine, tioguanine; Pyrimidine Analogs like azacitidine, capecitabine, carmofur, cytarabine, decitabine, fluorouracil, gemcitabine, tegafur; Vinca Alkaloids like vinblastine, vincristine, vindesine, vinflunine, vinorelbine; Podophyllotoxin Derivatives like etoposide, teniposide; Colchicine derivatives like demecolcine; Taxanes like docetaxel, paclitaxel, paclitaxel poliglumex; Other Plant Alkaloids and Natural Products like trabectedin; Actinomycines like dactinomycin; Antracyclines like aclarubicin, daunorubicin, doxorubicin, epirubicin, idarubicin, mitoxantrone, pirarubicin, valrubicin, zorubincin; Other Cytotoxic Antibiotics like bleomycin, ixabepilone, mitomycin, plicamycin; Platinum Compounds like carboplatin, cisplatin, oxaliplatin, satraplatin; Methylhydrazines like procarbazine; Sensitizers like aminolevulinic acid, efaproxiral, methyl aminolevulinate, porfimer sodium, temoporfin; Protein Kinase Inhibitors like dasatinib, erlotinib, everolimus, gefitinib, imatinib, lapatinib, nilotinib, pazonanib, sorafenib, sunitinib, temsirolimus; Other Antineoplastic Agents like alitretinoin, altretamine, amzacrine, anagrelide, arsenic trioxide, asparaginase, bexarotene, bortezomib, celecoxib, denileukin diftitox, estramustine, hydroxycarbamide, irinotecan, lonidamine, masoprocol, miltefosein, mitoguazone, mitotane, oblimersen, pegaspargase, pentostatin, romidepsin, sitimagene ceradenovec, tiazofurine, topotecan, tretinoin, vorinostat; Estrogens like diethylstilbenol, ethinylestradiol, fosfestrol, polyestradiol phosphate; Progestogens like gestonorone, medroxyprogesterone, megestrol; Gonadotropin Releasing Hormone Analogs like buserelin, goserelin, leuprorelin, triptorelin; Anti-Estrogens like fulvestrant, tamoxifen, toremifene; Anti-Androgens like bicalutamide, flutamide, nilutamide; Enzyme Inhibitors like aminoglutethimide, anastrozole, exemestane, formestane, letrozole, vorozole; Other Hormone Antagonists like abarelix, degarelix; Immunostimulants like histamine dihydrochloride, mifamurtide, pidotimod, plerixafor, roquinimex, thymopentin; Immunosuppressants like everolimus, gusperimus, leflunomide, mycophenolic acid, sirolimus; Calcineurin Inhibitors like ciclosporin, tacrolimus; Other Immunosuppressants like azathioprine, lenalidomide, methotrexate, thalidomide; and Radiopharmaceuticals like iobenguane.

In some embodiments, the anticancer agent is a toxin, e.g. diphtheria toxin. In certain embodiments, the biocompatible hydrogel polymer is loaded with a therapeutically effective amount of one or more toxins to form a biocompatible hydrogel polymer. Examples of toxins include Exotoxins like diphtheria toxin, botulinium toxin, cytolysins, hemolysins (e.g., α-toxin or α-hemolysin of *Staphyllococcus aureus),* cholera toxin, pertussis toxin, Shiga toxin; Heat-Stable Enterotoxin from *E. coli;* Curare; α-Cobratoxin; Verotoxin-1; and Adenylate Cyclase (AC) toxin from *Bordetella pertussis.*

In some cases, treatment comprises administration of a composition that specifically targets for degradation a nucleic acid sequence comprising a MEI-insertion adjacent contiguous sequence.

In addition to using an MEI border to select a treatment associated with a gene or gene product or pathway associated with a gene product tagged by the MEI insertion-adjacent sequence as discussed above, MEI-insertion border sequences are used in some cases to develop nucleic-acid targeting pharmaceuticals that directly target the sequence spanning the MEI and insertion-adjacent sequence. A number of compositions comprising nucleic acid sequence spanning MEI and insert adjacent border sequence are contemplated herein. In some cases, a common aspect of such compositions is that they comprise a nucleic acid component that is specific to a sequence spanning both the MEI edge sequence and insert-adjacent genomic sequence, and that is not sufficiently long to target either the MEI sequence or the insertion-adjacent sequence in isolation.

That is, the compositions contemplated and disclosed in many cases herein do not bind to the MEI in the absence of the insert-adjacent sequence, and do not bind to the insert adjacent sequence in the absence of an adjacent MEI; rather, the compositions disclosed herein comprise a nucleic acid component that specifically binds to a sequence comprising both an MEI and an adjacent genomic sequence. Thus, upon treatment with such a composition, only nucleic acids corresponding to a MEI-insert adjacent sequence, such as one that has been identified as disclosed herein to be substantially over-represented in a temporal or spatial assay as, for example, disclosed above, will be targeted by the composition, while other MEIs and uninserted alleles comprising the insert-adjacent sequence but not comprising the MEI sequence are not bound by the composition. In some cases a nucleic acid component of the composition comprises 3, 4, 5, 6, 7, 8, 9, 10, or more than 10 bases of MEI sequence and 3, 4, 5, 6, 7, 8, 9, 10, or more than 10 bases of the insert-adjacent sequence, such that the binding energy between the composition and the MEI alone or the composition and the insert-adjacent sequence alone is insufficient to secure binding.

Compositions as disclosed herein comprise, for example, a guide nucleic acid having characteristics as described above in combination with a moiety that directs endonucleolytic cleavage of a target sequence comprising the MEI and insertion-adjacent sequence.

In some embodiments, the guide nucleic acid molecule is a guide RNA molecule. In some cases the guide RNA molecule or other guide nucleic acid molecule directs endonucleolytic cleavage of the DNA molecule to which it is bound, for example by recruiting a protein having endonuclease activity such as Cas9 protein. Zinc Finger Nucleases (ZFN), Transcription activator like effector nucleases and Clustered Regulatory Interspaced Short palindromic Repeat /Cas based RNA guided DNA nuclease (CRISPR/Cas9), among others, are compatible with some embodiments of the disclosure herein.

A guide RNA molecule or other guide nucleic acid molecule comprises sequence that base-pairs with target sequence that is to be removed from sequencing (non-target sequence within the target sequence region). In some embodiments the base-pairing is complete, while in some embodiments the base pairing is partial or comprises bases that are unpaired along with bases that are paired to non-target sequence.

A guide RNA molecule or other guide nucleic acid molecule may comprise a region or regions that form a 'hairpin' structure. Such region or regions comprise partially or completely palindromic sequence, such that 5' and 3' ends of the region may hybridize to one another to form a double-strand 'stem' structure, which in some embodiments is capped by a non-palindromic loop tethering each of the single strands in the double strand loop to one another.

In some embodiments the guide RNA molecule or other guide nucleic acid molecule comprises a stem loop such as a tracrRNA stem loop. A stem loop such as a tracrRNA stem loop may complex with or bind to a nucleic acid endonuclease such as Cas9 DNA endonuclease. Alternately, a stem loop may complex with an endonuclease other than Cas9 or with a nucleic acid modifying enzyme other than an endonuclease, such as a base excision enzyme, a methyltransferase, or an enzyme having other nucleic acid modifying activity that interferes with one or more DNA polymerase enzymes.

The tracrRNA / CRISPR / Endonuclease system was identified as an adaptive immune system in eubacterial and archaeal prokaryotes whereby cells gain resistance to repeated infection by a virus of a known sequence. See, for example, Deltcheva E, Chylinski K, Sharma CM, Gonzales K, Chao Y, Pirzada ZA et al. (2011) "CRISPR RNA maturation by trans-encoded small RNA and host factor RNase III" Nature 471 (7340): 602-7. doi:10.1038/nature09886. PMC 3070239. PMID 21455174; Terns MP, Terns RM (2011) "CRISPR-based adaptive immune systems" Curr Opin Microbiol 14 (3): 321-7. doi:10.1016/j.mib.2011.03.005. PMC 3119747. PMID 21531607; Jinek M, Chylinski K, Fonfara I, Hauer M, Doudna JA, Charpentier E (2012) "A Programmable Dual-RNA-Guided DNA Endonuclease in Adaptive Bacterial Immunity" Science 337 (6096): 816-21. doi:10.1126/science.1225829. PMID 22745249; and Brouns SJ (2012) "A Swiss army knife of immunity" Science 337 (6096): 808-9. doi:10.1126/science.1227253. PMID 22904002. The system has been adapted to direct targeted mutagenesis in eukaryotic cells. See, e.g., Wenzhi Jiang, Huanbin Zhou, Honghao Bi, Michael Fromm, Bing Yang, and Donald P. Weeks (2013) "Demonstration of CRISPR/Cas9/sgRNA-mediated targeted gene modification in Arabidopsis, tobacco, sorghum and rice" Nucleic Acids Res. Nov 2013; 41(20): e188, Published online Aug 31, 2013. doi: 10.1093/nar/gkt780, and references therein.

As contemplated herein, a guide RNA molecule or other guide nucleic acid molecule are used in some embodiments to provide sequence specificity to a DNA endonuclease such as a Cas9 endonuclease. In these embodiments a guide RNA molecule or other guide nucleic acid molecule comprises a hairpin structure that binds to or is bound by an endonuclease such as Cas9 (other endonucleases are contemplated as alternatives or additions in some embodiments), and a guide RNA molecule or other guide nucleic acid molecule further comprises a recognition sequence that binds to or specifically binds to or exclusively binds to a sequence that is to be removed from a sequencing library or a sequencing reaction. The length of the recognition sequence in a guide RNA molecule or other guide nucleic acid molecule may vary according to the degree of specificity desired in the sequence elimination process. Nucleic acid specificity, as discussed above, is dictated by a requirement in many cases that the RNA molecule or other guide nucleic acid molecule bind specifically to an MEI-insertion adjacent sequence junction, but to neither the MEI nor the insertion-adjacent sequence alone. Short recognition sequences, comprising frequently occurring sequence in the sample or comprising differentially abundant sequence (abundance of AT in an AT-rich genome sample or abundance of GC in a GC-rich genome sample) are likely to identify a relatively large number of sites and therefore to direct frequent nucleic acid modification such as endonuclease activity, base excision, methylation or other activity that interferes with at least one DNA polymerase activity. Long recognition sequences, comprising infrequently occurring sequence in the sample or comprising underrepresented base combinations (abundance of GC in an AT-rich genome sample or abundance of AT in a GC-rich genome sample) are likely to identify a relatively small number of sites and therefore to direct infrequent nucleic acid modification such as endonuclease activity, base excision, methylation or other activity that interferes with at least one DNA polymerase activity. Accordingly, as disclosed herein, in some embodiments one may regulate the frequency of sequence removal from a sequence reaction through modifications to the length of the recognition sequence so as to target specifically a single MEI-insert adjacent sequence.

A guide RNA molecule or other guide nucleic acid molecule may be synthesized through a number of methods consistent with the disclosure herein. Standard synthesis techniques may be used to produce massive quantities of a guide RNA molecule or other guide nucleic acid molecule. The double stranded DNA molecules can comprise an RNA molecule or other guide nucleic acid molecule site specific binding sequence, a guide RNA molecule or other guide nucleic acid molecule sequence for Cas9 protein and a T7 promoter site. In some cases, the double stranded DNA molecules can be less than about 100bp length. T7 polymerase can be used to create the single stranded RNA molecules, which may include the target RNA sequence and a guide RNA sequence for the Cas9 protein.

Compositions as disclosed herein comprise, for example, a guide nucleic acid having MEI-insertion adjacent sequence binding characteristics as described above that directs silencing of a gene in the insertion adjacent sequence, such that a truncated or otherwise mutated allele of an gene product the insertion adjacent sequence, such as an oncogenic gene product, a gene product that causes a defect in cell cycle regulation, cell growth regulation or cell division regulation, for example, is silenced upon binding by the guide nucleic acid. In some cases the guide nucleic acid comprises a siRNA moiety, a piRNA moiety, or other nucleic acid moiety involved in gene silencing, transcriptional regulation or post-transcriptional regulation of a gene product.

siRNA and piRNA are small RNA molecules implicated in gene silencing. Introduction of dsRNA into an organism can cause specific interference of gene expression. This phenomenon, known as RNA interference (RNAi), results from a specific targeting of mRNA for degradation by cellular machinery in plant, invertebrate, and mammalian cells. Exemplary RNAi techniques known in the art include, without limitation, siRNA, shRNA and piRNA. Components of the RNAi machinery include the dsRNA targeting the target gene(s) (either siRNA or shRNA), Dicer, the Argonaute family of proteins (Ago-2 in particular), Drosha, RISC, TRBP, and PACT. Small interfering RNA (siRNA) is generally recognized as dsRNA with 2 nt 3' end overhangs that activate RNAi, leading to the degradation of mRNAs in a sequence-specific manner dependent upon complimentary binding of the target mRNA. shRNA is generally recognized as short hairpin RNA (shRNA) that contains a loop structure that is processed to siRNA and also leads to the degradation of mRNAs in a sequence-specific manner dependent upon complimentary binding of the target mRNA. Drosha is generally recognized as an RNase III enzyme that processes pri-miRNAs and shRNAs in the nucleus. Dicer is generally recognized as a ribonuclease (RNase) III enzyme which processes dsRNAs into 20-25 bp siRNAs leaving a 2 nt overhangs at the 3' end. *Drosophila* Dicer-2 cleaves long dsRNAs, while Dicer-1 is important for miRNA processing. RISC is generally recognized as the minimal RNA-induced silencing complex (RISC) consists of the Argonaute protein and an associated siRNA. It may also contain PACT, TRBP, and Dicer. It should be noted that the exact composition of RISC has yet to be described. TRBP is generally recognized as needed for dsRNA cleavage by Dicer and subsequent passage to the RISC. Protein R (PKR)-activating protein (PACT) is generally recognized as associating with Dicer and TRBP for dsRNA cleavage. Along with the single-stranded siRNA, argonaute family of proteins assemble to form the RISC, bind 21-35 nt RNAs including miRNAs and siRNAs, and their associated target mRNA and then cleaves them through its endonucleolytic function.

Small interfering RNA (siRNA), sometimes known as short interfering RNA or silencing RNA, is a class of double-stranded RNA molecules, generally 20-25 base pairs in length. siRNA is most notable in the RNA interference (RNAi) pathway, where it interferes with the expression of specific genes with complementary nucleotide sequences. siRNA functions by causing mRNA to be broken down after transcription, resulting in no translation. siRNA also acts in RNAi-related pathways, e.g., as an antiviral mechanism or in shaping the chromatin structure of a genome.

When choosing between siRNAs or shRNAs, an important factor to consider is the length of the treatment. siRNAs are transiently expressed in cells, while shRNAs can be stably integrated through virus-mediated transduction. Guidelines for siRNA design include: (1) siRNA sequences between 19-29 nt are generally recommended to avoid nonspecific silencing, (2) targeting sites which include AA dinucleotides and (3) siRNAs with 3' dUdU or dTdT dinucleotide overhangs enhance effectiveness. Generally, siRNA sequences should have a G/C content between 35-55%.

Protocols for delivery of the RNAi will depend on the cell type, since different cell types have varying sensitivities to the introduction of nucleic acids. Transfection, electroporation, and certain viral delivery methods are transient.

Among the most common nucleic acid delivery methods are transfection and electroporation. Transfection involves the formation of complexes of nucleic acids with carrier molecules that allow them to pass through the cell membrane. Transfection methods include lipid transfection, in which cationic lipids that have long hydrophobic chains with positively charged head groups interact with the negatively charged siRNA, surrounding it in a lipid bilayer, which is then endocytosed by the cell; cationic polymer-based nanoparticles, which allow for reduced toxicity and increased efficiency, as well as allowing for the delivery of modified siRNAs; and lipid or cell-penetrating peptide (CPP) conjugation, which involves conjugation of the siRNA with a hydrophobic moiety (e.g. cholesterol) or a cationic CCP (e.g. transportin or pentatratin), which promotes delivery into the target cells.

In electroporation methods, an electrical field is applied to the cell membrane, which is made up of phospholipid molecules with negatively charged head groups. The electrical pulse causes the phospholipids to reorient, creating pores in the membrane, allowing siRNAs to enter. Electroporation is commonly used for cells that are difficult to transfect. However, the specific settings (voltage, number of pulses, and length of the pulses) must be optimized for each cell or tissue type.

RNAi interventions are known to have therapeutic value for targeting cancers, neurological diseases, viral infections, macular degeneration, diabetic retinopathy, and hepatitis C, among other disorders.

Transposon silencing is a form of transcriptional gene silencing targeting transposons. Transcriptional gene silencing is a product of histone modifications that prevent the transcription of that area of DNA. Transcriptional silencing of transposons is crucial to the maintenance of a genome. The "jumping" of transposons generates genomic instability and can cause extremely deleterious mutations. Transposable element insertions have been linked to many diseases including hemophilia, severe combined immunodeficiency, and predisposition to cancer. The silencing of transposons is therefore extremely critical in the germline in order to stop transposon mutations from developing and being passed on to the next generation.

Piwi-interacting RNA (piRNA), the largest class of the small RNAs, are between 26 and 31 nucleotides in length and function through interactions with piwi proteins from the Argonaute protein family (gene silencing proteins). piRNAs bound to PIWI proteins are known in the art to use post-transcriptional transcript destruction to silence transposons. Most piRNAs are antisense to mRNAs transcribed from the silenced transposons, generally associating with Piwi and Aubergine (Aub) proteins, while sense-strand piRNAs tend to associate with Argonaute 3 (Ago3) instead. A cycle called "ping pong" amplification proceeds between the sense and anti-sense piRNAs involving extensive trimming and processing to create mature piRNAs. This process is responsible for the production of most piRNAs in the germline and could also explain the origin of piRNAs in germline development. Piwi-piRNA complexes repress transposon expression by increasing CpG methylation upstream or within the transposon region, and/or chromatin modification around transposon region, or by directly degrading a transposon's transcript.

Alternately or in combination, a treatment is selected in some cases associated with addressing cancers associated with misregulation of a cell growth, cell cycle or cell proliferation pathway for which the gene associated with the MEI encodes a participating member. For example, an MEI in a negative regulator of TOR (target of rapamycin signaling), such as a TSC2 locus, suggests treatment with a growth regulation inhibitor, while an MEI in a locus encoding the retinoblastoma tumor suppressor Rb suggests a treatment related to cell cycle progression.

In some cases, MEI levels are compared across locations in an individual or across time from a common sample source in an individual.

In some cases, blood is used as a source of nucleic acids to assay, such as free circulating nucleic acids, to be used in ongoing temporal monitoring of MEI levels, alone or in combination with alternate monitoring approaches. Alternately or in combination, circulating free DNA or other DNA from other sources are used in some embodiments.

Methods for extracting circulating free nucleic are known in the art. When nucleic acids are inside cells, procedures for extraction generally include cell lysis (commonly achieved by chemical and physical methods-blending, grinding or sonicating the sample), removing membrane lipids by adding a detergent or surfactant which also serves in cell lysis, optionally removing proteins by adding a protease, optionally removing RNA by adding an RNase (done when DNA is the desired target). Methods for DNA purification are known in the art. Exemplary DNA purification methods include, without limitation, ethanol precipitation, phenol-chloroform extraction, and mini-column purification. Ethanol precipitation can be done using ice-cold ethanol or isopropanol. Since DNA is insoluble in these alcohols, it will aggregate together, giving a *pellet* upon centrifugation. Precipitation of DNA is improved by increasing of ionic strength, usually by adding sodium acetate. Phenol-chloroform extraction denatures proteins in the sample. After centrifugation of the sample, denatured proteins stay in organic phase while aqueous phase containing nucleic acid is mixed with the chloroform that removes phenol residues from solution. For mini-column purification, the nucleic acid binds to a solid phase (silica or other) depending on the pH and the salt content of the buffer, and is then eluted.

Exemplary forms of circulating nucleic acid for extraction include, without limitation, DNA, RNA, mRNA, oligonucleosomal, mitochondrial, epigenetically modified, single-stranded, double-stranded, circular, plasmid, cosmid, yeast artificial chromosomes, artificial or man-made DNA, including unique DNA sequences, and DNA that has been reverse transcribed from an RNA sample, such as cDNA, and combinations thereof. Exemplary biological sources for extraction of nucleic acid include, without limitation, whole blood, serum, plasma, umbilical cord blood, chorionic villi, amniotic fluid, cerbrospinal fluid, spinal fluid, lavage fluid (e.g., bronchoalveolar, gastric, peritoneal, ductal, ear, athroscopic) biopsy sample, urine, feces, sputum, saliva, nasal mucous, prostate fluid, semen, lymphatic fluid, bile, tears, sweat, breast milk, breast fluid, embryonic cells and fetal cells. The biological sample can be any tissue or fluid that contains nucleic acids. Exemplary biological samples include, without limitation, paraffin imbedded tissue, frozen tissue, surgical fine needle aspirations, cells of the skin, muscle, lung, head and neck, esophagus, kidney, pancreas, mouth, throat, pharynx, larynx, esophagus, facia, brain, prostate, breast, endometrium, small intestine, blood cells, liver, testes, ovaries, uterus, cervix, colon, stomach, spleen, lymph node, bone marrow or kidney. Fluid samples may include bronchial brushes, bronchial washes, bronchial ravages, peripheral blood lymphocytes, lymph fluid, ascites, serous fluid, pleural effusion, sputum, cerebrospinal fluid, lacrimal fluid, esophageal washes, and stool or urinary specimens such as bladder washing and urine.

A nucleic acid sample source as discussed above or know in the art is obtained at a temporal interval or intervals, and nucleic acids are obtained for quantitative assessment of MEI insertion border abundances. Time points may be separated by days, weeks, months or years, such as 1 month, 2 months, 3 months, 4 months 5 months, 6months, 1 year, 2 years, three years, 4 years, 5 years, 10 years, or greater than 10 years.

In some cases time points are separated by partial or complete execution of a treatment regimen, such as excision of a tumor or other cancerous tissue, or administration of a treatment such as chemotherapy or radiotherapy targeted at eliminating the tumor or cancerous tissue. Treatment regimens and compositions as disclosed above are contemplated for use in the temporal analysis of a treatment regimen in some cases.

Thus, MEI level quantification for an MEI associated with hyper-proliferative cells is used, for example, to monitor the efficacy of the intervention, wherein a decrease in the level of the MEI indicates efficacy, or a decrease in the rate of increase in the relative level of the MEI indicates efficacy, or a stabilization of the relative amount of the MEI insertion border at a steady level indicates efficacy.

Spatial rather than temporal separation of samples is also contemplated herein. Thus, in some cases samples are taken from a first region or tissue not phenotypically associated with tumor or cancer activity, and a second sample is taken from a second region or tissue suspected of cancerous activity or precancerous activity, or observed to be a tumor or cancer.

In some cases samples are taken from a plurality of regions within a cancer or tumor, such as quiescent and mitotically active or proliferatively active regions, such that cells associated with tumor proliferation, growth, cell division, or metastisis are separated from cells associated with benign, quiescent or senescent tumor tissue.

In some cases tumor tissues are distinguished spatially, such that, for example, interior and edge cell populations are separately extracted. Alternately or in combination, tumor cells are sorted by surface characteristics or biomarkers.

Several methods for cell sorting are known in the art. Exemplary types of cell sorting include, without limitation, fluorescent activated cell sorting (FACS), magnetic cell selection and single cell sorting. Single cell sorting provides a method for sorting a heterogeneous mixture of cells based upon intracellular and extracellular properties. FACS utilizes flow cytometry to provide quantitative measurement of intra- and extracellular properties, not including morphology, for sorting a heterogeneous mixture of cells. Magnetic cell sorting provides a method for enriching a heterogeneous mixture of cells based upon extracellular properties, typically cell-surface proteins (i.e., antigens). Magnetic-activated cell sorting (MACS) is a column based separation technique where labeled cells are passed through a magnetic column. SEP system provides a column-free cell separation technique in which a tube of labeled cells is placed inside a magnetic field. Positively selected cells are retained in the tube while negatively selected cells are in the liquid suspension. Methods of cell sorting include sorting agents (e.g., antibodies) that specifically bind cancer biomarkers to sort cells.

Exemplary cancer biomarkers include, without limitation, CCR10, CD9, CD13, CD15, CD24, CD26, CD29, CD32, CD46, CD49a, CD49b, CD49c, CD49f, CD51, CD54, CD55, CD56, CD58, CD63, CD66a, CD66c, CD66e, CD71, CD73, CD81, CD82, CD91, CD98, CD99, CD102, CD104, CD105, CD108, CD111, CD117, CD118, CD130, CD131, CD133, CD136, CD141, CD146, CD147, CD148, CD151, CD155, CD157, CD164, CD166, CD167a, CD172a, CD177, CD186, CD196, CD221, CD230, CD234, CD244, CD245, CD262, CD265, CD273, CD275, CD295, CD298, CD299, CD317, CD318, CD324, CD340, BMPR-1B, cadherin-11, c-Met, Claudin-3, DLL-1, DLL-3, Eph-B2, Eph-B4, FOLR1, Frizzled-3, Glut-1, Glut-2, Glypican 5, HLA-A/B/C, HLA-A2, HER3, IL-15R, IL-20Ra,jagged-2, integrin-a8, integrin a9b1, integrin b5, LAG-3, leukotriene-B4R, Lox-1, LDL-R, MCSP, mer, nectin-4, notch2, NPC, PD-L2, Plexin-B1, semaphorin 4B, somatostatin-R2, TROP-2, ULBP2, integrin aVb9 and VEGFR2. In the case of single-cell-sorting and FACS, biomarkers can be intracellular or extracellular.

MEI levels are compared across samples to identify MEI insertion junctions that are differentially overabundant in the second sample. As discussed herein a differentially abundant MEI insertion junction is in some cases 10%, 20%, 30%, 40%, 50%, 70%, 100%, 2x, 2.5x, 3x, 3.5x, 4x, 5x or greater than 5x more abundant in one sample rather than another.

MEI insertion borders so identified as differentially present in putatively unhealthy tissue are used to guide treatment selection as discussed above. MEI insertion borders so identified as differentially present in putatively unhealthy tissue are used to monitor disease progression or treatment efficacy, such that a decrease in relative levels, or a stabilization of relative levels, or a reduced rate of increase of relative levels, indicates treatment efficacy.

In some cases, MEI-insertion adjacent sequences associated with hyper-proliferative cellular activity are used to monitor for tumor or cancer or precancerous cell expansion beyond an identified tumor or cancer site, such that an increase in the relative abundance of the MEI insertion site in a sample derived from a putatively healthy tissue is indicative of a risk that the tissue from which the sample is derived is potentially precancerous or cancerous.

A report detailing results of an MEI quantitative sequencing analysis is provided in some embodiments. The report comprises information regarding MEI relative abundance levels over a time course, relative to a treatment regiment, or in one tissue or region relative to another, for example. In some cases the report is accompanied by treatment recommendations related to or informed by the identity of the sequence adjacent to the MEI insertion site or sites associated with hyper-proliferative cells. Such treatment recommendations comprise in various embodiments chemotherapy, radiotherapy, tissue excision, or combinations thereof. In some cases the treatment targets a product of the disrupted gene associated with the MEI insertion site, while in some embodiments the treatment targets misregulation of a member of a pathway in which the product of the disrupted gene participates. For example, if a negative regulator is disrupted as indicated by a MEI insertion, a treatment may target a downstream signaling component which is expected to be upregulated as a result of the MEI insertion disruption.

The report is provided to the individual in some cases, while in some cases the report is provided to a health care professional. Reports are in some cases provided in confidence, such that they are not provided to the public but are directed only to the individual providing the samples or the individual and an associated healthcare professional, or confidentially provided to a health care professional.

A number of methods are available for MEI-insertion adjacent sequence quantification. A conceptual example of how the repetitive elements such as MEI sequences are quantitatively assayed through whole genome sequencing is as follows.

Sequence information obtained herein is used in some cases to nucleic acid sequence abundance in a sample. A library is generated and sequenced as disclosed herein or as known in the art. Duplicate reads are excluded so that only uniquely tagged reads are included. Unique read sequences are mapped to a genomic sequence. The number of unique library sequence reads mapping to a target region is counted and is used to represent the abundance of that sequence in the sample. In some embodiments uniquely tagged sequence reads each map to a single site in the sample sequence. In some cases, uniquely tagged sequence reads map to a plurality of sites throughout a genome, such as transposon insertion sites or repetitive element sites. Accordingly, in some cases the number of library molecules mapping to a transcriptome 'locus' or transcript corresponds to the level of accumulation of that transcript in the sample from which the library is generated. The number of library molecules mapping to a repetitive element, relative to the number of library molecules that map to a given unique region of the genome, is indicative of the relative abundance of the repetitive element in the sample. Sequence reads mapping to a given MEI insertion junction are used to quantify that insertion junction in a given sample. Thus, by comparing the number of reads spanning an MEI insertion border, one quantifies that insertion border relative to, for example, other sequence in the sample, such as sequence known to be single copy in a healthy haploid genome of the sample.

Thus, quantifying the relative abundance of a nucleic acid molecule sequence in a sample is effected by generating a sequence library comprising uniquely tagged library fragments and mapping the nucleic acid molecule sequence onto the library, such as the frequency of occurrence of the nucleic acid molecule sequence in the library corresponds to the abundance of the nucleic acid molecule sequence in the sample from which the library is generated. In some cases the frequency of occurrence of the nucleic acid molecule sequence in the library is assessed relative to the frequency of occurrence of a second nucleic acid molecule sequence in the library, said second nucleic acid sequence corresponding to a locus or transcript of known abundance in a transcriptome or known copy number per genome of a genomic sample.

A more detailed protocol for nucleic acid sequence quantification in a nucleic acid sample is provided below. It is emphasized, however, that the methods disclosed herein are not limited to any single method of nucleic acid sequence quantification in a nucleic acid sample.

Generating Next Generation Sequencing (NGS) libraries from every possible position in a genome requires an unbiased approach to converting genomic DNA (gDNA) template into the appropriate size library molecule with the platform specific sequencing adapters flanking the gDNA. This may be performed using a random primer with a sequencing adapter tail, as illustrated by the following schematic: 5'-adapter sequence-NNNNNNNN-3'.

To minimize bias for a given genome, the "random" portion of the primer may be synthesized in a semi-random fashion to account for variable content in the genome of interest. A given genome *(e.g.,* the human genome) can be broken up into 100bp windows of varying GC content. Ideally, primers would be synthesized to include representative "randomness" ordered against the windows of GC content in the genome from 1% to 100% GC and synthesized and pooled in ratios relative to the content of the genome at each GC%.

Random priming can allow for each base of a genome to be represented as the start position for a sequencer read. In order to end each library molecule at every possible base in the genome, a random/unbiased approach to terminate polymerization from a random primer is required. To do this, a cocktail of ddNTPs containing a fixed ratio of each of the four native nucleotides to a fixed ratio of dideoxynucleotides that are devoid of a 3'-OH group may be used. The ratio of ddNTP to dNTP can determine the probability of termination at any given base position. For example, a 1% ddNTP cocktail (99% dNTP) would give a probability that 99% of molecules extending from a random primer will polymerize past the first base. This same example would give a N50 (50% of the molecules will be longer than N bases) of 50bp. As the relative ddNTP proportion decreases, the N50 insert size increases. Thus, under certain conditions, a ddNTP% of 0.8 leads to a median insert size (N50) of 62.5, and a comparable N50 of full length library molecules including adapters and random primers of 198.5, a ddNTP% of 0.4 leads to a median insert size (N50) of 125 and a comparable N50 of full length library molecules including adapters and random primers of 261, a ddNTP% of 0.2 leads to a median insert size (N50) of 250 and a comparable N50 of full length library molecules including adapters and random primers of 386, a ddNTP% of 0.1 leads to a median insert size of 500 and a comparable N50 of full length library molecules including adapters and random primers of 636, and a ddNTP% of 0.05 leads to a median insert size of 1000 and a comparable N50 of full length library molecules including adapters and random primers of 1136. For regions of low complexity, such as stretches of AT or GC, the effective concentration of ddNTP in that genomic location would be reduced by half, giving an N50 of 100 nucleotides for a primer extension reaction occurring in such low complexity genomic loci with a 1% ddNTP cocktail. (Not accounting for polymerase incorporation efficiency differences amongst all 8 nucleotides).

Adjusting the ddNTP % in the reaction can adjust the range and diversity of the polymerized molecules. The effect of the ddNTP concentration on fragment length and adenine-tyrosine bias is shown in FIG. 11. The effect of ddNTP concentration on yield is shown in FIG. 12. At 0.4% ddNTP, the molarity from 300-1000bp (mole) is 27.5; at 0.2% ddNTP, the molarity from 300-1000bp (mole) is 16.1; at 0.1% ddNTP, the molarity from 300-1000bp (mole) is 5.8; and at 0.05% ddNTP, the molarity from 300-1000bp (mole) is 4.9. FIG. 13 shows the read position for molecules selected by size.

An additional step can be to isolate the adapter-labeled molecules from the gDNA template and any excess reactants such as primers and excess NTPs. This can be done through the use of biotinylated ddNTPs. A streptavidin coated magnetic bead can be used to accomplish this isolation.

The choice of polymerase can be restricted to an enzyme that has the capabilities of strand displacement as well as ddNTP/biotin incorporation. SEQUENASE and THERMOSEQUENASE (Affymetrix, Santa Clara, CA) are two such enzymes. If low input amounts are required due to lack of sample resource or forced dilution, the reaction may be optimized to improve yield through the use of enzyme cocktails such as SEQUENASE and Phi29, a highly processive polymerase devoid of the ability to incorporate ddNTPs. The phi 29 enzyme will increase the template amount for processing by SEQUENASE in the reaction. The yield and diversity of template may also be increased by optimizing the duration of the reaction.

The product of such a sequencing reaction is represented by the following schematic: 5'-ADAPTER-NNNNNNNN-GENOMIC INSERT-ddNTP/biotin.

Current commercial sequencers require the gDNA insert to be flanked by 2 adapter sequences. The second adapter may be added through a second random priming reaction. The isolated product from the magnetic beads can be used as template for a second random priming reaction using a random primer with a second adapter, as demonstrated by the schematic: 5'-Adapter2-NNNNNNNN-3'. The displaced product may also be used as template for a second random priming reaction using a random primer with a second adapter.

The enzyme for the second adapter addition may not require the ability to incorporate ddNTP. Strand displacement may be a requirement. Acceptable enzymes include SEQUENASE, THERMOSEQUENASE, Phi29, *Bst* DNA Polymerase, and *Taq* DNA polymerase. The random portion of the primer can bind to the bead bound template and extend through the end of the template molecule. The primer that binds closest to the 3' end of the template can displace the primers that are bound downstream so that a single copy of the bead bound template will be produced with both the first and second adapters. This copy can remain hydrogen-bonded to the magnetic beads. Excess primer, NTP, enzyme and displaced product can be removed through bead washing. The resulting product can be heat denatured (releasing it from the bead) and sequenced or amplified through PCR with primers complementary to the adapters. A product created thereby is represented by the following schematic, depicted in 3' to 5' orientation: 3'-adapter1-NNNNNNNN-gDNA insert-NNNNNNNN-adapter2-5'.

A critical error mode in NGS sequencing is the clonal amplification of errors in the library prep. For PCR free protocols this may be less of a concern, but any low input protocol requires amplification to obtain enough library to load on a sequencer. Errors introduced in the amplification process may show up in a sequencer. A standard reduction in these errors is to remove duplicates from analysis. However, if enough sequencing capacity is given to a sample, duplicate reads (reads with the same start and end position) may occur naturally. Removing these reads would therefore reduce coverage and accuracy of the assay. The use of the synthetic random primers in analysis can allow for a true determination of clonal artifacts vs low frequency mutations. PCR duplicates may have the same random primer sequences on both ends while duplicates due to deep sequencing coverage may have different random primer sequences. Since the synthetic sequence is always at the same position of each read, this information can be easily obtained in the analysis.

Non terminating sequencing by synthesis chemistries (such as Qiagen and ION Torrent) experience difficulty sequencing long stretches of homopolymers. This may be mitigated by the complex library generation achieved through termination at each base across the homopolymer described herein.

Accordingly, consistent with the disclosure above, first strand oligonucleotide libraries are generated. To generate a Random Library, a population of first round synthesis oligos is synthesized. The first strand oligonucleotides each comprise a sequence adapter positioned 5' of a random oligomer sequence, such as a 2, 3, 4, 5, 6, 7, 8, 9, 10, 11, 12, 13, 14, 15, 16, 17, 18, 19, 20, 21, 22, 23, 24, 25, 26, 27, 28, 29, 30 mer, or larger oligomer, followed by a 3' OH from which template directed extension occurs. In some cases the sequence adapter is configured to comprise variable identifier sequence. In alternate cases, the sequence adapter is invariant. Sequence adapters are in some cases used as primer binding sites for the later addition of a sequencing adapter, such as an A adapter, such as through standard primer-directed sequence addition through amplification.

In some cases the oligonucleotide population is synthesized such that all possible combinations of a given random oligomer base sequence (such as random 5, 6, 7, 8, 9, or 10 mers) are represented in the first strand oligonucleotide population. In other cases, particularly when a long random oligomer is selected, but also occasionally in cases of smaller oligomers, less than all possible combinations of a given random oligomer base sequence are present.

In some cases the bases of the random oligomer represent an unbiased random distribution of nucleic acid bases in equal proportions. In some cases each base is equally likely to occur at a given position, or in aggregate in a random oligomer population. In other cases, however, to increase the efficiency of annealing and, subsequently, first strand synthesis, the population is synthesized so as to include a bias for random oligomers (such as random 8 mers) having a biased representation of certain bases or base pairs. The human genome, for example, is observed to have a GC percentage of about 40%, rather than a 50% GC composition as expected from a true random base abundance. See, for example FIG. 10. In some cases the random oligomer distribution is biased such that the overall distribution of random oligomer sequence (such as 8 mer sequence) in the first strand synthesis library reflects that of a skewed target average, such as the average of a target genome, a target locus, a target gene family, a target genomic element (such as exons, introns, or promoter sequence, for example), or in some embodiments, to match the human genome as a whole.

A first strand oligo library or a subset of an oligonucleotide library representing 90%, 80%, 70%, 60%, 50%, 40%, 30%, 20%, 10%, or less than 10% of a first strand oligonucleotide library is contacted to a sample comprising a nucleic acid such as deoxyribonucleic acid or ribonucleic acid. A nucleic acid such as DNA or RNA may be provided in a wide range of amounts. In some cases a genomic DNA sample is provided at or about an amount such as 1ng, 2ng, 3ng, 4ng, 5ng, 6ng, 7ng, 8ng, 9ng, 10ng, 11ng, 12ng, 13ng, 14ng, 15ng, 16ng, 17ng, 18ng, 19ng, 20ng, 21ng, 22ng, 23ng, 24ng, 25ng, 26ng, 27ng, 28ng, 29ng, 30ng, 31ng, 32ng, 33ng, 34ng, 35ng, 36ng, 37ng, 38ng, 39ng, 40ng, 41ng, 42ng, 43ng, 44ng, 45ng, 46ng, 47ng, 48ng, 49ng, 50ng, 51ng, 52ng, 53ng, 54ng, 55ng, 56ng, 57ng, 58ng, 59ng, 60ng, 61ng, 62ng, 63ng, 64ng, 65ng, 66ng, 67ng, 68ng, 69ng, 70ng, 71ng, 72ng, 73ng, 74ng, 75ng, 76ng, 77ng, 78ng, 79ng, 80ng, 81ng, 82ng, 83ng, 84ng, 85ng, 86ng, 87ng, 88ng, 89ng, 90ng, 91ng, 92ng, 93ng, 94ng, 95ng, 96ng, 97ng, 98ng, 99ng or 100ng, or a value outside of the range defined by the above-mentioned list. As seen below, the number of downstream thermocycles will decrease as the amount of starting template increases. In some cases an RNA sample is provided from RNA extracted from a cell population of as few as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 cells, or more than 100 cells.

Also added to the mixture is a polymerase buffer comprising reagents consistent with DNA polymerase activity. A number of polymerases are consistent with the disclosure herein. In some cases, exemplary polymerases possess strand displacement activity, ddNTP incorporation activity, and are able to incorporate biotin-labeled nucleotides such as biotin-labeled ddNTP. An exemplary polymerase is Sequenase, while an exemplary reverse-transcriptase is HIV reverse-transcriptase.

Also added to the mixture is a population of nucleotides, such as a population comprising dATP, dTTP, dCTP and dGTP, and in some cases also comprising a population of ddNTP, such as ddATP, ddTTP, ddCTP and ddGTP. In some cases only a single species of ddNTP is added to the population of dNTP, such as ddATP alone, ddTTP alone, ddCTP, alone, and ddGTP alone. In some cases ddNTP pairs are added, such as ddATP and ddTTP, or ddCTP and ddGTP.

In some cases, the population of ddNTP, such as ddATP, ddTTP, ddCTP and ddGTP added to the composition comprises at least one biotin tagged ddNTP, such as biotin tagged ddATP, biotin tagged ddTTP, biotin tagged ddCTP and biotin tagged ddGTP.

A range of dNTP / ddNTP ratios are consistent with the disclosure herein. Ratios of 99.9% / 0.1%, 99.5% / 0.5%, 99% / 1%, 98% / 2% and alternate ratios are consistent with the disclosure herein. In some cases a relative ratio of 99% deoxy NTP to 1% dideoxy NTP is selected.

The mixture is denatured, in some cases by heating above a melting temperature, such as 95°C, 96°C, 97°C, 98°C or 99°C, or a higher temperature. In many cases a denaturing temperature below 100°C is exemplary.

The mixture is then cooled, for example on ice for 30 seconds, 1, 2, or more than 2 minutes, or at 4°C for 30 seconds, 1, 2, or more than 2 minutes, or at an alternate cooling temperature, sufficient to allow for reverse-complementary base-pairing between the first strand synthesis oligonucleotides and the nucleic acid sample such as a genomic DNA sample or an RNA sample. In some cases some or all of the first strand synthesis oligonucleotides demonstrate complete reverse-complementarity between their random oligo (such as a random 8 mer) and the nucleic acid sample sequence such as genomic DNA sequence, cDNA sequence or RNA sequence, to which each binds. In some cases, some oligonucleotides bind to genomic regions that are incompletely reverse-complementary to the oligo's random oligomer (such as a random 8 mer). The failure to base pair with complete reverse complementarity in some cases is not detrimental to subsequent steps in the random library prep process.

A polymerase is added before or after an optional denaturing step in alternate embodiments. The mixture is heated to a temperature consistent with polymerase activity, such as optimal polymerase activity (for example, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, or in some cases a number greater or less than a number in this range), and incubated for a period sufficient to synthesize the first strand library, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or more than 45 minutes. In some cases the reaction is agitated at points during this incubation, such as every 10 minutes.

Extension progresses from the 3' OH of the first strand synthesis oligonucleotides, resulting in sequence reverse complementary to the template at the annealing site of each annealed oligo being incorporated at the 3' end of each annealed oligo. Extension continues until a biotin-labeled ddNTP molecule is incorporated, at which point extension terminates. If dNTP and biotin-ddNTP are provided at a ratio of 99% / 1%, 50% of the first strand oligos on which extension occurs demonstrate an extension of over 50 bases prior to the incorporation of a biotin-ddNTP molecule. In some cases where other parameters are not simultaneously varied, the proportion of ddNTP decreases, the N50, representing the length of at least 50% of the extension products, increases.

At the completion of the incubation period the reaction is stopped, for example by heat inactivation at 98°C for five minutes. Alternately, inactivation may be accomplished at another temperature, or by addition of a chelating agent or a dNTPase.

As mentioned above, in some cases an incorporated ddNTP is tagged, such as by a biotin tag. Alternatives to biotin are contemplated in some cases, such as dinitrophenyl. Any affinity tag that can be bound to ddNTP and incorporated into a nascent nucleic acid molecule by at least one nucleic acid polymerase is consistent with the disclosure herein. Similarly, any affinity tag that can be delivered to a ddNTP end of a nucleic acid molecule, for example via a ddNTP binding moiety, is also consistent with the disclosure herein. In some cases the affinity tag is biotin -ddNTP.

In some cases a tag-binding agent is provided to bind to tagged first strand nucleic acid molecules as provided herein, such as avidin or streptavidin in the case of the tag biotin. In particular cases the streptavidin is bound to magnetic beads, such that streptavidin and any binding partner can be isolated by placement in a magnetic field, such as on a magnetic stand.

Tagged first strand libraries are isolated using a tag-binding agent, for example streptavidin against a biotin tagged ddNTP nucleic acid end. In some cases the bead / sample mixture is incubated at 22C and agitated at 10 minute intervals for 30 minutes. The mixture is then put on a magnetic stand and, upon settling of the beads, the supernatant is removed. The tube is agitated and allowed to settle on a magnetic stand. Beads are washed three times with 200uL of TE buffer. Alternative tag-binding agent combinations and alternative protocols are consistent with the disclosure herein.

In some cases, first strand molecules are purified independent of tagging, for example by size selection, such as gel electrophoresis, followed by purification of nucleic acids of a desired size. In some cases fragments of a size range of 10-100, 10-150, 10-200, 1-300, 10-350, 10-400, 10-500, 10-600, 10-700, 10-800, 10-900, or 10-1000, bases are isolated.

First strand library templates as purified above are reintroduced into a reaction buffer. For example, templates are in some cases separated from their purification tags, eluted from the streptavidin tags and resuspended in nucleic acid synthesis buffer including dNTP. In some cases, templates remain attached to their purification tags, are washed, and resuspended in reaction buffer. A NaOH wash is included following first strand library generation in some cases, to remove carryover sequences and to decrease self-folding of the first strand library product.

Library second strand molecules are synthesized as follows. A second probe library is added, comprising a population of second strand primers. In some cases each second strand primer comprises a B-adapter sequence 5' to a random oligomer sequence such as a 2, 3, 4, 5, 6, 7, 8, 9, 10, 11, 12, 13, 14, 15, 16, 17, 18, 19, 20, 21, 22, 23, 24, 25, 26, 27, 28, 29, 30 mer, or larger oligomer (for example an 8 mer) followed by a 3' OH from which template directed extension occurs. In some cases the sequence adapter is configured to comprise variable identifier sequence. In alternate cases, the sequence adapter is invariant. Sequence adapters are in some cases used as primer binding sites for the later addition of a sequencing adapter, such as a B adapter, such as through standard primer-directed sequence addition through amplification.

In some cases then oligonucleotide population is synthesized such that all possible combinations of a given random oligomer base sequence (such as random 8 mers) are represented in the second strand oligonucleotide population. In other cases, particularly when a long random oligomer is selected, but also occasionally in cases of smaller oligomers, less than all possible combinations of a given random oligomer base sequence are present.

In some cases the bases of the random oligomer represent an unbiased random distribution of nucleic acid bases in equal proportions. In some cases each base is equally likely to occur at a given position, or in aggregate in a random oligomer population. In other cases, however, to increase the efficiency of annealing and, subsequently, second strand synthesis, the population is synthesized so as to include a bias for random oligomers (such as random 8 mers) having a biased representation of certain bases or base pairs. The human genome, for example, is observed to have a GC percentage of about 40%, rather than a 50% GC composition as expected from a true random base abundance. See, for example FIG. 10. In some cases the random oligomer distribution is biased such that the overall distribution of random oligomer sequence (such as 8 mer sequence) in the second strand synthesis library reflects that of a skewed target average, such as the average of a target genome, a target locus, a target gene family, a target genomic element (such as exons, introns, or promoter sequence, for example), or in some embodiments, to match the human genome as a whole.

The mixture is heated to 98°C for 3 minutes. The mixture is cooled on ice for 2 minutes allow for reverse-complementary base-pairing between the second strand synthesis oligonucleotides and the first strand library. It is observed that some oligonucleotides demonstrate complete reverse-complementarity between their random 8 mer and the first strand sequence to which each binds. It is also observed that some oligonucleotides bind to genomic regions that are incompletely reverse-complementary to the oligo's random 8 mer. The failure to base pair with complete reverse complementarity is not detrimental to subsequent steps in the random library prep process.

The composition is heated to room temperature and allowed to continue for 30 minutes. For samples with lower amount of input DNA, this time period can be lengthened.

Extension from the 3' OH of the first strand synthesis oligonucleotides is observed, resulting in sequence reverse complementary to the template at the annealing site of each annealed oligo being incorporated at the 3' end of each annealed oligo. Extension continues until the 5' end of the first strand template is reached. It is observed that second-strand oligos annealing away from the 3' end of the first strand template undergo extension from their 3' ends, but are displaced from the first strand by extension reactions primed by oligos annealing further toward the 3' end of the first strand template.

Accordingly, double-stranded library molecules are synthesized, comprising two distinct strands: 1) a first strand having, from the 5' end, an A adapter, a random 8 mer sequence and target sequence on the order of 1-100 nucleotides, terminating in a biotin-tagged ddNTP; and 2) a second strand having, from the 5' end a B adapter, a second random 8 mer sequence, a target sequence derived from the sample, a first random 8 mer sequence reverse complementary to the random 8 mer of the first strand, and sequence reverse complementary to the first A adapter.

In some cases, magnetic streptavidin beads are used to isolate the biotin-tagged double-stranded library molecules. Magnetic streptavidin bead are provided, for example, in binding buffer, mixed, and allowed to settle on a magnetic stand. The binding buffer may then be replaced to a 25uL, 50uL, 75uL, 100uL, 125uL, 150uL, 175uL, 200uL, 225uL, 250uL, 275uL, 300uL, 350uL, 400uL, 450uL, or 500uL volume and the process repeated. The supernatant is then drawn off and the beads may be resuspended in 5uL, 10uL, 12uL, 14uL, 16uL, 18uL, 20uL, 22uL, 24uL, 26uL, 28uL, 30uL, 31uL, 32uL, 33uL, 34uL, 35uL, 36uL, 37uL, 38uL, 39uL, 40uL, 41uL, 42uL, 43uL, 44uL, 45uL, 46uL, 47uL, 48uL, 49uL 50uL, 52uL, 54uL, 56uL, 58uL, or 60uL of binding buffer.

In some cases, the biotin-tagged double-stranded library molecules are then added to the resuspended beads. In some cases, the bead / sample mixture is incubated at 22C and agitated at 10 minute intervals for 30 minutes. The mixture is then put on a magnetic stand and, upon settling of the beads, the supernatant is removed. The tube is agitated and allowed to settle on a magnetic stand. Beads are washed three times with 200uL of TE buffer. In some cases, this results in a population of streptavidin purified, double-stranded library molecules, comprising two distinct strands: 1) a first strand having, from the 5' end, an A adapter, a random oligomer (such as an 8 mer) sequence and target sequence on the order of 1-100 nucleotides, terminating in a biotin-tagged ddNTP; and 2) a second strand having, from the 5' end a B adapter, a second random oligomer (such as an 8 mer) sequence, a target sequence derived from the sample, a first random oligomer (such as an 8 mer) sequence reverse complementary to the random oligomer (such as an 8 mer) of the first strand, and sequence reverse complementary to the first A adapter. Alternative tag-binding agent combinations and alternative protocols are consistent with the disclosure herein.

The magnetic streptavidin beads bound to the population of double-stranded library molecules are then, for example, resuspended in an amount of nuclease-free water. This amount may be 10uL, 12uL, 14uL, 16uL, 18uL, 20uL, 22uL, 24uL, 26uL, 28uL, 30uL, 32uL, 34uL, 36uL, 37uL, 38uL, 39uL, 40uL, 41uL, 42uL, 43uL, 44uL, 45uL, 46uL, 47uL, 48uL, 50uL, 52uL, 54uL, 56uL, 58uL, or 60uL of nuclease-free water. An amount of Adapter A primer and an amount of Adapter B primer is added to the resuspended beads. The amount of Adapter A primer and the amount of Adapter B primer may be the same or they may be different. The amount of Adapter A primer and the amount of Adapter B primer may independently be 1uL, 2uL, 3uL, 4uL, 5uL, 6uL, 7uL, 8uL, 9uL, or 10uL. In some cases, the Adapter A primer comprises sequence identical to the first adapter of the double-stranded template at the primer's 3' end, and further comprises sequence necessary for sequencing by synthesis reactions as described herein. In other cases, the Adapter A primer has one base-pair mismatch, two base-pair mismatches, three base-pair mismatches, four base-pair mismatches, five base-pair mismatches, six base-pair mismatches, seven base-pair mismatches, eight base-pair mismatches, nine base-pair mismatches, or ten base-pair mismatches with the sequence of the first adapter of the double-stranded template at the primer's 3' end. In some cases, Adapter B primer comprises sequence identical to the second adapter of the second strand of the double-stranded template at the primer's 3' end, and further comprises sequence necessary for sequencing by synthesis reactions as described herein. In other cases, the Adapter B primer has one base-pair mismatch, two base-pair mismatches, three base-pair mismatches, four base-pair mismatches, five base-pair mismatches, six base-pair mismatches, seven base-pair mismatches, eight base-pair mismatches, nine base-pair mismatches, or ten base-pair mismatches with the sequence of the second adapter of the second strand of the double-stranded template at the primer's 3' end.

2x PCR master mix is added in an amount of 10uL, 15uL, 20uL, 25uL, 30uL, 35uL, 40uL, 45uL, 50uL, 55uL, 60uL, 65uL, 70uL, 75uL, 80uL, 85uL, 90uL, 95uL, or 100uL to the mixture of beads and primers. In some cases, this mixture is then subjected to thermocycling as follows: about 98°C for about 2 minutes; followed by about 6 cycles of about 98°C, for about 20 second, about 60°C, for about 30 seconds, and about 72°C, for about 30 seconds; following said about six cycles the reaction is held at about 72°C for about 5 minutes and then is stored at about 4°C. Optimization of the thermocycling conditions is envisioned by the instant disclosure, such as increasing the number of PCR cycles for samples with lower template input. In some cases, amplification is performed without PCR. In an example, template nucleic acid is used with primers containing full length sequencing adapters and first strand synthesis and second strand synthesis is performed with a subsequent size selection. This may or may not require the use of hairpins to avoid dimerization.

In some cases, the sequencing library generated thereby is observed to have the following characteristics. Each double-stranded molecule comprises, in order, an adapter A sequence sufficient for sequencing by synthesis, a first random oligomer sequence (such as an 8 mer), a target region of unknown length but likely within 1-100 bases, a second random oligomer (such as an 8 mer) sequence, and a B adapter sequence sufficient for sequencing by synthesis as disclosed herein.

In some cases, it is observed that the library constituents possess the following characteristics. Each molecule comprises a first molecular tag (such as an 8 mer) that is independent of the first molecular tag (such as an 8 mer) of other molecules in the library. Each molecule comprises a target sequence, corresponding to sequence of the original sample. The starting point of the target sequence, the length of the target sequence, and the endpoint of the target sequence of each given molecule is independent of the starting point, length and end point of each other molecule in the library. Each molecule comprises a second molecular tag (such as an 8 mer) that is independent of the second molecular tag (such as an 8 mer) of other molecules in the library.

In some cases, it is observed that the library, in aggregate, possesses the following characteristics. Substantially all of the sample sequence is represented in the library by multiple overlapping molecules. Substantially all of the library molecules (barring rare events), prior to the final addition of A and B adapters through thermocycling, are unique, varying from one another as to their first molecular tag (such as an 8 mer) sequence, target sequence starting point, target sequence, target sequence length, target sequence end point, and second molecular tag (such as an 8 mer) sequence.

A sequence library as generated herein is subjected to sequence by synthesis compatible with its A adapter and B adapter, and the sequence results are assessed. Independently, a second aliquot of the original sample is prepared for sequencing using standard PCR-based library tagging involving substantial PCR-based amplification of untagged template. The libraries are sequenced and the results compared.

It is observed that a sequence corresponding to an MEI is identified in the traditional sequence library sequencing results. The ME monomer unit is observed to be found adjacent to multiple insertion-adjacent border sequences, suggesting that it is present in multiple copies in the sample.

As the sequence reads are uniquely tagged by a 5' tag, a 3' tag, and a unique starting pint, end point and length of the sample sequence in each library member, sequence reads are easily sorted into groups corresponding to unique library molecules. By counting the number of unique library molecules represented in the sequence read population rather than the number of sequence reads, one can obtain a quantitative measurement of the absolute or relative number of molecules having a given MEI-insertion-adjacent sequence in a nucleic acid sample subject to sequencing.

Alternative quantification approaches are available, and the methods disclosed herein are not limited by a single method of quantification. For example, quantitative PCR is used in some cases to determine MEI-insertion adjacent sequence levels in a sample or samples.

Generally, quantitative PCR is carried out in a thermal cycler with the capacity to illuminate each sample with a beam of light of a specified wavelength and detect the fluorescence emitted by the excited fluorophore. The thermal cycler is also able to rapidly heat and chill samples, thereby taking advantage of the physicochemical properties of the nucleic acids and DNA polymerase. The PCR process generally consists of a series of temperature changes that are repeated 25 - 40 times. These cycles normally consist of three stages: the first, at around 95 °C, allows the melting of the double-stranded nucleic acid; the second, at a temperature of around 50-60 °C, allows the binding of the primers with the DNA template; the third, at between 68 - 72 °C, facilitates the polymerization carried out by the DNA polymerase. Due to the small size of the fragments the last step is usually omitted in this type of PCR as the enzyme is able to increase their number during the change between the alignment stage and the denaturing stage. In addition, some thermal cyclers add another short temperature phase lasting only a few seconds to each cycle, with a temperature of, for example, 80 °C, in order to reduce the noise caused by the presence of primer dimers when a non-specific dye is used. The temperatures and the timings used for each cycle depend on a wide variety of parameters, such as: the enzyme used to synthesize the DNA, the concentration of divalent ions and dNTPs in the reaction and the bonding temperature of the primers.

In the case of quantitative PCR (qPCR), a DNA-binding dye binds to double-stranded (ds) DNA in PCR, causing fluorescence of the dye. An increase in DNA product during PCR leads to an increase in fluorescence intensity and is measured at each cycle, thus allowing DNA concentrations to be quantified. Quantitative PCR can also include fluorescent reporter probes to detect only the DNA containing the probe sequence, which increases specificity and enables quantification even in the presence of non-specific DNA amplification.

Methods of quantification using qPCR include relative quantification and absolute quantification. Absolute quantification gives the exact number of target DNA molecules by comparison with DNA standards using a calibration curve. Relative quantification is based on internal reference genes to determine fold-differences in expression of the target gene. The quantification is expressed as the change in expression levels of mRNA interpreted as complementary DNA (cDNA, generated by reverse transcription of mRNA).

Unlike end point PCR (conventional PCR) real time PCR allows quantification of the desired product at any point in the amplification process by measuring fluorescence. A commonly employed method of DNA quantification by quantitative PCR relies on plotting fluorescence against the number of cycles on a logarithmic scale. A threshold for detection of DNA-based fluorescence is set slightly above background. The number of cycles at which the fluorescence exceeds the threshold is called the threshold cycle (Cₜ) or quantification cycle (C_{q}).

Commercial quantitative PCR compositions, kits and methods are available, and their use is consistent with some methods disclosed herein relating to MEI-insert adjacent sequence quantification.

Some embodiments disclosed herein relate to the monitoring of general somatic genomic health over time. General genomic health, as disclosed herein, relates to somatic genome 'health' status as reflected by the abundance of independent MEI events, in some cases independent of insertion site. Thus in some cases methods relate to the temporal or spatial assaying of the total number of MEI events. In some cases an increase in the number of MEI events indicates a decrease in 'aggregate genomic health,' as each insertion event conveys a risk of harm to an associated insertion site gene. The aggregate number of MEI events is in some cases correlated with a risk for cancer, senescence, loss of cellular activity, or reduction in cellular activity.

Aggregate MEI events are determined, for example using quantitative whole genome sequencing as disclosed herein or elsewhere. Alternately or in combination, individual mobile elements are assayed using, for example, Q-PCR or a fluorescence in situ-hybridization approach, as known in the art, using primers, probes or primers and probes specific to a single mobile element, or using panels of primers, probes or primers and probes such that a plurality of mobile elements, up to and including 10%, 20%, 30%, 40%, 50%, 60%, 70%, 80%, 90%, 95%, or about 100%, or 100% of known mobile elements, are quantified as to their abundance at a first time point or in a first tissue.

This quantification is used in some cases as a baseline for genome health, particularly if the sample is taken from a tissue or at a first time period when genomic health is expected to be high, such as in youth or early adulthood.

A second sample is taken at a second time point, such as a time point less than 1, 1, 2, 3, 4, 5, 10, or more than 10 years after the first time point. Aggregate MEI levels are measured and comparted to levels at the initial time point, or levels objectively associated with genomic health for patients generally.

The nucleic acids in the sample are determined to be 'senscent' or in poor genomic health if the aggregate number of MEI events has increased by 10%, 20%, 30%, 40%, 50%, 70%, 100%, 2x, 2.5x, 3x, 3.5x, 4x, 5x or greater than 5x more abundant in the second sample rather than the first or a previous sample. A number of treatment options are available for an individual determined to have somatic nucleic acid sample in poor genomic health. In some cases caloric restriction is selected. In some cases NSAIDS are recommended as part of a treatment regimen. A partial list of NSAIDs includes the following: aspirin, celecoxib (Celebrex), diclofenac (Cambia, Cataflam, Voltaren-XR, Zipsor, Zorvolex), diflunisal, etodolac, ibuprofen (Motrin, Advil), indomethacin (Indocin), ketoprofen, ketorolac, nabumetone, naproxen (Aleve, Anaprox, Naprelan, Naprosyn), oxaprozin (Daypro), piroxicam (Feldene), salsalate, sulindac, and tolmetin. Other NSAIDs are contemplated and are consistent with the disclosure herein.

Mobile element activity is associated with retrotransposase activity and with defects in repressive genome methylation in some cases. Thus, in some cases a treatment regimen comprises administration of a reverse transcriptase inhibitor. In some cases treatment comprises administration of a retrotransposase inhibitor. In some cases treatment comprises administration of a retroviral inhibitor. Treatment methods may be administered based on information obtained from genomic analysis. Treatment regimens for genetic abnormalities are known in the art. Exemplary inhibitors administered to treat a retroviral disorder include, without limitation, nucleoside analogues, protease inhibitors, non-nucleoside invert transcriptase inhibitors (NNRTIs), nucleotide slow transcriptase inhibitors (NtRTIs), blend inhibitors or entry inhibitors, and integrase inhibitors. Exemplary NRTIs include zidovudine (Retrovir), lamivudine (Epivir), didanosine (Videx), zalcitabine (Hivid), stavudine (Zerit) and abacavir (Ziagen). Exemplary protease inhibitors include saquinavir (Invirase), ritonavir (Norvir), indinavir (Crixivan), nelfinavir (Viracept), amprenavir (Agenerase), lopinavir, atazanavir (Reyataz) and tipranavir (Aptivus). Exemplary non-nucleoside invert transcriptase inhibitors (NNRTIs) include nevirapine (Viramune), delavirdine (Rescriptor), efavirenz (Sustiva) and etravirine (Intelence). Exemplary NtRTIs include tenofovir (Viread). Exemplary blend inhibitors or entry inhibitors include Maraviroc and Enfuvirtide. Exemplary integrase inhibitors include Raltegravir (Isentress). Alternately or in combination with any combinaiton of the above-listed treatments, a methyl-transferase or DNA methylation-promoting composition is administered to the individual. Exemplary inhibitors to treat HBV include, without limitation, interferon alpha (IFN-α), PEG-IFN-α, entecavir and tenofovir.

In some cases treatment is monitored over time as to its effect upon the increase in MEI abundance. For example, a third sample is taken at a time point subsequent to the initiation of a treatment regimen such as a treatment regimen disclosed herein, such as a time point of 1 month, 2 months, 3 months, 4 months, 5 months, 6 months, 7 months, 8 months, 9 months, 10 months, 11 months, less than 1, 1, 2, 3, 4, 5, 10, or more than 10 years after the first time point. Aggregate MEI levels are measured and compared to levels at the initial time point, or levels objectively associated with genomic health for patients generally, or levels determined prior to initiation of a treatment regimen, or are compared to a prior MEI abundance measurement. Treatment regimens that result in a decrease in the rate of increase in MEI abundance up to and including a stabilization of MEI aggregate amounts at pre-insertion levels are continued, in some cases accompanied by ongoing monitoring of aggregate MEI levels. Treatment regimens that do not impact total aggregate MEI level increase are replaced, supplemented, or dosage regimens are modified or increased such that MEI level increases are likely to be positively impacted.

In some cases this assay is performed in combination with monitoring of specific MEI insertion adjacent sites that demonstrate a specific increase over time, or with monitoring of MEI adjacent borders to identify events that involve a known or suspected oncogene such as an oncogene as listed herein or a genomic rearrangement associated with oncogenic activity such as a genomic rearrangement as listed herein, or both, such that MEI insertion events particularly suspected of being associated with current or future cancer or tumor activity are identified early and addressed, for example using compositions and methods disclosed herein.

For cellular health, a test or tests are performed at an early age and monitored in the blood for cell free DNA of insertional events. An increase in the same insertional events represents clonal expansion of the event and can be quantified and associated with disease progression. The test may be used in combination with tissue specific testing for MEI insertions, with germline variant analysis including exome or whole genome sequencing or with methylation or quantitative RNA analysis to determine cell health or progression of disease.

In addition, some embodiments of the disclosure herein relate to the visualization of tissue having a MEI insertion border, such as a MEI insertion related border associated with hyper-proliferation such as that in cancer or in a tumor cell population. In some cases an oligonucleotide probe is used, having nucleotide sequence that specifically anneals to a nucleic acid sequence comprising a MEI-insertion adjacent contiguous sequence, such that upon annealing the probe is detectable, for example to a medical practitioner assaying for successful excision of cancerous or tumor tissue.

MEI-insertion border sequences are used in some cases to develop nucleic-acid targeting probes that directly visualize the sequence spanning the MEI and insertion-adjacent sequence. A number of compositions comprising nucleic acid sequence spanning MEI and insert adjacent border sequence are contemplated herein. In some cases, a common aspect of such compositions is that they comprise a nucleic acid component that is specific to a sequence spanning both the MEI edge sequence and insert-adjacent genomic sequence, and that is not sufficiently long to target either the MEI sequence or the insertion-adjacent sequence in isolation.

That is, the compositions contemplated and disclosed in many cases herein do not bind to the MEI in the absence of the insert-adjacent sequence, and do not bind to the insert adjacent sequence in the absence of an adjacent MEI; rather, the compositions disclosed herein comprise a nucleic acid component that specifically binds to a sequence comprising both an MEI and an adjacent genomic sequence. Thus, upon treatment with such a composition, only nucleic acids corresponding to a MEI-insert adjacent sequence, such as one that has been identified as disclosed herein to be substantially over-represented in a temporal or spatial assay as, for example, disclosed above, will be visualized by the composition, while other MEIs and uninserted alleles comprising the insert-adjacent sequence but not comprising the MEI sequence are not bound by the composition. In some cases a nucleic acid component of the composition comprises 3, 4, 5, 6, 7, 8, 9, 10, or more than 10 bases of MEI sequence and 3, 4, 5, 6, 7, 8, 9, 10, or more than 10 bases of the insert-adjacent sequence, such that the binding energy between the composition and the MEI alone or the composition and the insert-adjacent sequence alone is insufficient to secure binding.

Also bound to the nucleic acid in some embodiment is a fluorophore or other visualizeable moiety. In some cases the moiety is visualized only when the nucleic acid is bound to a substrate. For example, a probe comprises in some cases a flouorophore and a quenching agent, such that in the absence of binding to a target MEI insertion-adjacent site, the quenching moiety prevents fluorescence, but in the presence of binding to a target MEI insertion-adjacent site, the quenching agent is spatially removed from the fluorophore such that the fluorophore is capable of emission upon excitation with an excitation agent.

The probe is in some cases used to assay for complete excision of cancerous tissue. Tissue is excised and contacted with a probe. Cancerous tissue is confirmed by the presence of fluorescence in the excised tissue, for example upon being subject to a wavelength of electromagnetic energy compatible with the excitation spectrum of the fluorophore. Noncancerous tissue is identified by an absence of fluorescence upon being subject to a wavelength of electromagnetic energy compatible with the excitation spectrum of the fluorophore. A number of excitation devices are known in the art, such as hand-held excitation devices that are readily used in an operating room environment.

It is known in the art that chemically reactive derivatives of a fluorophores and other dyes can be used as reporters for labeling molecules. Exemplary DNA binding reporters include, without limitation: SeTau-380-NHS, Hydroxycoumarin, Aminocoumarin, Methoxycoumarin, Cascade Blue, Pacific Blue, Pacific Orange, SeTau-405-NHS, SeTau-405-Maleimide, Lucifer yellow, SeTau-425-NHS, NBD, R-Phycoerythrin (PE), Seta-PerCP-680, PE-Cy5 conjugates, PE-Cy7 conjugates, Red 613, PerCP, TruRed, FluorX, Fluorescein, BODIPY-FL, Cy2, Cy3, Seta-555-NHS, Seta-555-Azide, Seta-555-DBCO, Seta-R-PE-670, Cy3B, Seta-580-NHS, Cy3.5, SeTau-647-NHS, Cy5, Seta-APC-780, Cy5.5, Seta-680-NHS, Cy7, TRITC, X-Rhodamine, Lissamine Rhodamine B, Texas Red, Allophycocyanin (APC), APC-Cy7 conjugates, an Seta-780-NHS.

Fluorophores and other reporters can be used to bind to probes which bind DNA. Such probes are known in the art to be designed to increase the specificity of quantitative PCR. For example, the TaqMan probe principle relies on the 5' to 3' exonuclease activity of *Taq* polymerase to cleave a dual-labeled probe during hybridization to the complementary target sequence and fluorophores-based detection. The resulting fluorescence signal permits quantitative measurements of the accumulation of the product during the exponential stages of the PCR.

TaqMan probes consist of a fluorophore covalently attached to the 5'-end of the oligonucleotide probe and a quencher at the 3'-end. Additional probes with different chemistries are known in the art and include, without limitation, 6-carboxyfluorescein or tetrachlorofluorescein, and quenchers (e.g. tetramethylrhodamine). A quencher molecule quenches fluorescence emitted by a fluorophore when excited by a thermocycler's light source via FRET (Fluorescence Resonance Energy Transfer). As long as the fluorophore and the quencher are in proximity, quenching inhibits fluorescence signals.

In some cases the probe comprises a moiety that directs the translocation of the probe across a cell membrane, across a nuclear membrane, or both a cell membrane and a nuclear membrane, such that access to tissue nuclear DNA is facilitated.

In addition, some embodiments disclosed herein relate to the identification of a biological sample such as a human sample other animal sample, plant sample, or biohazard sample by comparing its profile of MEI insertion adjacent sequences to that of a second sample or known reference profile. A sample for which a profile is to be determined is subjected to a process of MEI insertion-adjacent sequence determination, for example by whole genome sequencing or other appropriate method, and its individual MEI insertion adjacent profile is determined. In some cases a primer panel, probe panel, or primer panel and probe panel is developed so that the sample's MEI insert-adjacent sequence profile is detected in other samples without reliance upon whole genome sequencing.

A sample of unknown origin is obtained, of the same species and phenotype as the sample for which an MEI insertion-adjacent profile has been developed. In some cases the sample is of a crop plant such as a transgenic crop plant, and there is some question as to the origin of the crop plant germ line. A profile of a commercially sold transgenic plant of the same species and having the same transgenic resistance is obtained, and compared to the MEI insertion adjacent profile of the sample of unknown origin. By comparing the MEI insertion-adjacent sequence of the sample and the reference, one determines whether the sample and the reference are from a recent common stock.

In alternate embodiments, MEI insertion adjacent profiles are used to determine the origin of a forensic sample, for example, or a biohazard material such as anthrax, Yersinia pestris, methicillin-resistant Staphylococcus aureus (MRSA) or other weaponizable biological material.

In some embodiments identifying a second nucleic acid sample as different from a first or reference nucleic acid sample comprises determining whether said second nucleic acid sample lacks an MEI border sequence present in the first nucleic acid sample.

In some embodiments identifying said second nucleic acid sample as different from said first nucleic acid sample comprises determining whether said second nucleic acid sample includes an MEI border sequence not present in said first nucleic acid sample.

Border sequences are determined by targeted sequencing or by whole genome sequencing or both in alternate embodiments. In some cases a sample is contacted with a probe such as the probes discussed above, or a panel of probes, and sample identification is effected in some cases by assessment of the florescence of the sample upon probe excitation, individually, in series or in combination, upon contacting with probe molecules.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EXAMPLES

**Example 1. Temporal MEI monitoring.** A nucleic acid sample from an individual is subjected to whole genome quantitative sequencing. MEI insertion sites are identified that occur at a frequency of once per two haploid genome copies, indicating that the event generating the MEI insert likely occurred in the individual's ancestral germ line rather than in the individual's somatic cells.

MEI insertion sites are identified that occur at a frequency of less than once per two haploid genome copies, indicating that the events have occurred in some but not all somatic cells of the individual. MEI insertion sites are examined, and it is determined that some MEI insertion sites are likely to have disrupted genes for which loss of function is associated with defects in cell cycle regulation, cell growth regulation, or cell division regulation.

MEI insertion site abundance is monitored over time. After two years, a nucleic acid sample from the individual in Example 1 is taken from the individual's blood. Nucleic acids from the individual's blood are assayed.

MEI insertion sites are identified. It is observed that a first MEI insertion site occurs at a frequency comparable to the frequency observed in the previous whole genome sequencing effort. The MEI insertion border is concluded not to be associated on its own with a defect in cell cycle regulation, cell growth regulation, or cell division regulation.

It is observed that a second MEI insertion site occurs at a frequency that is 10x higher that the frequency observed in the previous whole genome sequencing effort. The MEI insertion border is concluded to be associated with a defect in cell cycle regulation, cell growth regulation, or cell division regulation. The individual is subjected to further observation to look for cancer or other under-regulated cell proliferation defect from which DNA can be obtained to determine whether the tumor or other cell defect corresponds with the MEI insertion border.

A putatively cancerous tissue is identified. A nucleic acid sample from the putatively cancerous tissue is subjected to whole genome quantitative sequencing. The second MEI insertion site occur is found to occur at a frequency that is 100x that of the frequency in the original whole genome MEI survey.

**Example 2. Temporal MEI monitoring.** A nucleic acid sample from the individual in Example 1 is taken from the individual's blood. Nucleic acids from the individual's blood are assayed, and relative and absolute MEI insertion site frequencies are determined.

The putatively cancerous tumor tissue is excised from the individual. Following the procedure, a second nucleic acid sample from the individual in Example 1 is taken from the individual's blood. Nucleic acids from the individual's blood are assayed, and relative and absolute MEI insertion site frequencies are determined. It is observed that the frequency of the second MEI insertion site has returned to the frequency in the original whole genome MEI survey.

**Example 3. Temporal MEI monitoring.** A nucleic acid sample from the individual in Examples 1 and 2 is taken from the individual's blood two years after excision of the putatively cancerous tumor tissue. Nucleic acids from the individual's blood are assayed, and relative and absolute MEI insertion site frequencies are determined. It is observed that the frequency of the second MEI insertion site remains at the frequency in the original whole genome MEI survey.

A nucleic acid sample from the individual in Examples 1 and 2 is taken from the individual's blood four years after excision of the putatively cancerous tumor tissue. Nucleic acids from the individual's blood are assayed, and relative and absolute MEI insertion site frequencies are determined. It is observed that the frequency of the second MEI insertion site is 5x above the frequency in the original whole genome MEI survey.

The individual is subjected to further observation to look for cancer or other under-regulated cell proliferation defect from which DNA can be obtained to determine whether the tumor or other cell defect corresponds with the MEI insertion border.

A putatively cancerous tissue is identified. A nucleic acid sample from the putatively cancerous tissue is subjected to whole genome quantitative sequencing. The second MEI insertion site occur is found to occur at a frequency that is 100x that of the frequency in the original whole genome MEI survey.

The putatively cancerous tumor tissue is excised from the individual. Following the procedure, a nucleic acid sample is taken from the individual's blood. Nucleic acids from the individual's blood are assayed, and relative and absolute MEI insertion site frequencies are determined. It is observed that the frequency of the second MEI insertion site has returned to the frequency in the original whole genome MEI survey.

**Example 4. Spatial MEI monitoring.** A first nucleic acid sample from phenotypically healthy tissue from an individual suffering from a tumor is subjected to whole genome quantitative sequencing. MEI insertion sites are identified that occur at a frequency of less than once per two haploid genome copies, indicating that the events have occurred in some but not all somatic cells of the individual. MEI insertion sites are examined, and it is determined that some MEI insertion sites are likely to have disrupted genes for which loss of function is associated with defects in cell cycle regulation, cell growth regulation, or cell division regulation.

A second nucleic acid sample from tumor tissue from an individual suffering from a tumor is subjected to whole genome quantitative sequencing. MEI insertion sites are identified that occur at a frequency of less than once per two haploid genome copies, indicating that the events have occurred in some but not all tumor cells of the individual. MEI insertion sites are examined, and it is determined that some MEI insertion sites are likely to have disrupted genes for which loss of function is associated with defects in cell cycle regulation, cell growth regulation, or cell division regulation.

Relative and absolute abundances of insertion sites are examined. It is observed that some MEI insertion sites occur at relative and absolute frequencies comparable to those found in the individual's phenotypically healthy-derived nucleic acid sample. It is concluded that these sites are not related to defects in cell cycle regulation, cell growth regulation, or cell division regulation

MEI sites unique to the tumor tissue nucleic acid sample are identified. Some tumor-specific MEI insertion sites occur at low abundance in tumor tissue nucleic acid samples. It is concluded that these MEI insertions are not correlated with tumor activity.

Some MEI insertion sites are found throughout the tumor tissue nucleic acid sample. It is concluded that these MEI insertion sites are prerequisite for the manifestation of defects in cell cycle regulation, cell growth regulation, or cell division regulation. However, their relatively abundant presence in non-tumor nucleic acid samples indicates that they do not on their own indicate the presence of defects in cell cycle regulation, cell growth regulation, or cell division regulation associated with tumor activity.

Some MEI insertion sites are found at a very high frequency throughout the tumor tissue nucleic acid sample, and are found at a very low frequency in the non-tumor nuclei acid sample. It is concluded that these MEI insertion sites are indicative of the manifestation of defects in cell cycle regulation, cell growth regulation, or cell division regulation associated with tumor activity.

**Example 5. Specific MEI insertion border targeting**. The MEI insertion border from Examples 2-3 is used as a source for pharmaceutical intervention. A nucleic acid molecule comprising MEI insertion sequence and insertion-adjacent genomic sequence is developed. The molecule is packaged into a CRISPR nucleic acid-targeting complex that specifically directs an endonuclease to cleave nucleic acids adjacent to the MEI insertion sequence and insertion-adjacent genomic sequence, and that does not cleave other MEI insertion sites.

**Example 6**. **Therapeutic intervention to deplete cells having MEI insertion borders associated with putative cancerous tissue**. A nucleic acid sample from the individual in Examples 1 and 2 is taken from the individual's blood two years after excision of the putatively cancerous tumor tissue. Nucleic acids from the individual's blood are assayed, and relative and absolute MEI insertion site frequencies are determined. It is observed that the frequency of the second MEI insertion site remains at the frequency in the original whole genome MEI survey.

A nucleic acid sample from the individual in Examples 1 and 2 is taken from the individual's blood four years after excision of the putatively cancerous tumor tissue. Nucleic acids from the individual's blood are assayed, and relative and absolute MEI insertion site frequencies are determined. It is observed that the frequency of the second MEI insertion site is 5x above the frequency in the original whole genome MEI survey.

The individual is subjected to further observation to look for cancer or other under-regulated cell proliferation defect from which DNA can be obtained to determine whether the tumor or other cell defect corresponds with the MEI insertion border.

A putatively cancerous tissue is identified. A nucleic acid sample from the putatively cancerous tissue is subjected to whole genome quantitative sequencing. The second MEI insertion site occur is found to occur at a frequency that is 100x that of the frequency in the original whole genome MEI survey.

The individual is treated with a treatment regimen comprising the MEI insertion border-targeting pharmaceutical of Example 5. The putatively cancerous tissue is observed to undergo specific cell death.

Following the procedure, a nucleic acid sample is taken from the individual's blood. Nucleic acids from the individual's blood are assayed, and relative and absolute MEI insertion site frequencies are determined. It is observed that the frequency of the second MEI insertion site has returned to the frequency in the original whole genome MEI survey.

**Example 7. Therapeutic intervention to deplete cells having MEI insertion borders associated with putative cancerous tissue**. A nucleic acid sample from the individual in Examples 1 and 2 is taken from the individual's blood two years after excision of the putatively cancerous tumor tissue. Nucleic acids from the individual's blood are assayed, and relative and absolute MEI insertion site frequencies are determined. It is observed that the frequency of the second MEI insertion site remains at the frequency in the original whole genome MEI survey.

A nucleic acid sample from the individual in Examples 1 and 2 is taken from the individual's blood four years after excision of the putatively cancerous tumor tissue. Nucleic acids from the individual's blood are assayed, and relative and absolute MEI insertion site frequencies are determined. It is observed that the frequency of the second MEI insertion site is 5x above the frequency in the original whole genome MEI survey.

The individual is subjected to further observation to look for cancer or other under-regulated cell proliferation defect from which DNA can be obtained to determine whether the tumor or other cell defect corresponds with the MEI insertion border.

No putatively cancerous tissue is identified.

The individual is treated with a treatment regimen comprising the MEI insertion border-targeting pharmaceutical of Example 5.

Following the procedure, a nucleic acid sample is taken from the individual's blood. Nucleic acids from the individual's blood are assayed, and relative and absolute MEI insertion site frequencies are determined. It is observed that the frequency of the second MEI insertion site has returned to the frequency in the original whole genome MEI survey.

**Example 8. Monitoring of age-specific genome senescence**. A nucleic acid sample from an individual is subjected to whole genome quantitative sequencing. MEI insertion sites are identified that occur at a frequency of once per two haploid genome copies, indicating that the event generating the MEI insert likely occurred in the individual's ancestral germ line rather than in the individual's somatic cells.

MEI insertion sites are identified that occur at a frequency of less than once per two haploid genome copies, indicating that the events have occurred in some but not all somatic cells of the individual. MEI insertion sites are examined, and it is determined that some MEI insertion sites are likely to have disrupted genes for which loss of function is associated with defects in cell cycle regulation, cell growth regulation, or cell division regulation.

MEI insertion site abundance is monitored over time. After five years, a nucleic acid sample from the individual is taken from the individual's blood. Nucleic acids from the individual's blood are assayed.

MEI insertion sites are observed to occur with a relative frequency and with relative abundances comparable to those observed following initial whole genome quantitative sequencing.

After ten years, a nucleic acid sample from the individual is taken from the individual's blood. Nucleic acids from the individual's blood are assayed.

MEI insertion sites are observed to occur with relative abundances comparable to those observed following initial whole genome quantitative sequencing. However, novel MEI insertion events are observed to have occurred, raising the total number of insertion sites by 2x.

An anti-aging regimen comprising caloric restriction is recommended.

After 15 years, a nucleic acid sample from the individual is taken from the individual's blood. Nucleic acids from the individual's blood are assayed.

MEI insertion sites are observed to occur with relative abundances comparable to those observed at ten years, indicating that the increase in MEI insertion site frequency has not continued.

**Example 9. Monitoring of age-specific genome senescence**. A nucleic acid sample from an individual is subjected to whole genome quantitative sequencing. MEI insertion sites are identified that occur at a frequency of once per two haploid genome copies, indicating that the event generating the MEI insert likely occurred in the individual's ancestral germ line rather than in the individual's somatic cells.

MEI insertion sites are identified that occur at a frequency of less than once per two haploid genome copies, indicating that the events have occurred in some but not all somatic cells of the individual. MEI insertion sites are examined, and it is determined that some MEI insertion sites are likely to have disrupted genes for which loss of function is associated with defects in cell cycle regulation, cell growth regulation, or cell division regulation.

MEI insertion site abundance is monitored over time. After five years, a nucleic acid sample from the individual is taken from the individual's blood. Nucleic acids from the individual's blood are assayed.

MEI insertion sites are observed to occur with a relative frequency and with relative abundances comparable to those observed following initial whole genome quantitative sequencing.

After ten years, a nucleic acid sample from the individual is taken from the individual's blood. Nucleic acids from the individual's blood are assayed.

MEI insertion sites are observed to occur with relative abundances comparable to those observed following initial whole genome quantitative sequencing. However, novel MEI insertion events are observed to have occurred, raising the total number of insertion sites by 2x.

An anti-aging regimen comprising treatment with a reverse-transcriptase inhibitor is followed.

After 15 years, a nucleic acid sample from the individual is taken from the individual's blood. Nucleic acids from the individual's blood are assayed.

MEI insertion sites are observed to occur with relative abundances comparable to those observed at ten years, indicating that the increase in MEI insertion site frequency has not continued.

**Example 10. Monitoring of age-specific genome senescence**. A nucleic acid sample from an individual is subjected to whole genome quantitative sequencing. MEI insertion sites are identified that occur at a frequency of once per two haploid genome copies, indicating that the event generating the MEI insert likely occurred in the individual's ancestral germ line rather than in the individual's somatic cells.

MEI insertion sites are identified that occur at a frequency of less than once per two haploid genome copies, indicating that the events have occurred in some but not all somatic cells of the individual. MEI insertion sites are examined, and it is determined that some MEI insertion sites are likely to have disrupted genes for which loss of function is associated with defects in cell cycle regulation, cell growth regulation, or cell division regulation.

MEI insertion site abundance is monitored over time. After five years, a nucleic acid sample from the individual is taken from the individual's blood. Nucleic acids from the individual's blood are assayed.

MEI insertion sites are observed to occur with a relative frequency and with relative abundances comparable to those observed following initial whole genome quantitative sequencing.

After ten years, a nucleic acid sample from the individual is taken from the individual's blood. Nucleic acids from the individual's blood are assayed.

MEI insertion sites are observed to occur with relative abundances comparable to those observed following initial whole genome quantitative sequencing. However, novel MEI insertion events are observed to have occurred, raising the total number of insertion sites by 2x.

An anti-aging regimen comprising treatment with a retrovirus inhibitor is followed.

After 15 years, a nucleic acid sample from the individual is taken from the individual's blood. Nucleic acids from the individual's blood are assayed.

MEI insertion sites are observed to occur with relative abundances comparable to those observed at ten years, indicating that the increase in MEI insertion site frequency has not continued.

The invention includes the following aspects:
1. A method of identifying mobile element insertion (MEI) tagged cell proliferation comprising the steps of
   quantitatively measuring MEI levels at a first MEI insertion site in a first nucleic acid sample;
   quantitatively measuring MEI levels at a first MEI insertion site in a second nucleic acid sample; and
   identifying the first MEI insertion site as tagging MEI tagged cell proliferation if MEI levels at a first MEI insertion site in a first nucleic acid sample differ substantially from MEI levels at a first MEI insertion site in a second nucleic acid sample.
2. The method of aspect 1, wherein said first nucleic acid sample and said second nucleic acid sample comprise substantially similar amounts of nucleic acids.
3. The method of any one of aspects 1-2, wherein a control nucleic acid is present at substantially similar amounts in the first nucleic acid sample and the second nucleic acid sample.
4. The method of any one of aspects 1-3, comprising identifying the sequence adjacent to the first MEI insertion site.
5. The method of aspect 4, comprising selecting a treatment associated with efficacy in addressing a defect in the sequence adjacent to the first MEI insertion site.
6. The method of any one of aspects 1-5, wherein the first nucleic acid sample and the second nucleic acid sample are obtained from a common individual at a first time point and a second time point.
7. The method of aspect 6, wherein said first time point and second time point are separated by a treatment administered to said individual.
8. The method of aspect 7, wherein the treatment comprises cancer therapy.
9. The method of aspect 6, wherein said first time point and second time point are separated by at least 6 months.
10. The method of aspect 6, wherein said first time point and second time point are separated by at least 1 year.
11. The method of aspect 6, wherein said first time point and second time point are separated by at least 2 years.
12. The method of aspect 6, wherein said first time point and second time point are separated by at least 5 years.
13. The method of any one of aspects 1-12, wherein said first nucleic acid sample and said second nucleic acid sample are extracted from blood.
14. The method of any one of aspects 1-13, wherein said first nucleic acid sample and said second nucleic acid sample comprise circulating free nucleic acids.
15. The method of any one of aspects 1-14, wherein said first nucleic acid sample and said second nucleic acid sample comprise circulating free genomic DNA.
16. The method of any one of aspects 1-5, wherein the first nucleic acid sample is obtained from an individual at a first location and the second nucleic acid sample is obtained from the individual at a second location.
17. The method of aspect 16, wherein the first location comprises a first cancerous tissue.
18. The method of any one of aspects 16-17, wherein the second location comprises healthy tissue.
19. The method of any one of aspects 16-17, wherein the second location comprises a second cancerous tissue.
20. The method of aspect 19, wherein the second cancerous tissue and the first cancerous tissue are derived from a common cancer.
21. The method of any one of aspects 1-20, comprising generating a report disclosing said MEI levels at a first MEI insertion site in a first nucleic acid sample and said MEI levels at a first MEI insertion site in a second nucleic acid sample.
22. The method of aspect 21, wherein said report is provided to said individual.
23. The method of aspect 21, wherein said report is provided to a health care professional.
24. The method of aspect 21, wherein said report is made confidentially.
25. A Mobile Element Insertion (MEI) monitoring regimen comprising the steps of obtaining genome sequence information from an individual comprising a plurality of MEI insertion borders; reviewing the plurality of MEI insertion borders to identify a border adjacent to an oncogene; and monitoring the quantitative abundance of the MEI border adjacent to the oncogene over time.
26. The method of aspect 25, wherein said monitoring the quantitative abundance of the MEI border adjacent to the oncogene over time comprises obtaining a first blood sample at a first time point, determining the quantitative abundance of the MEI border in the first blood sample at the first time point, obtaining a second blood sample at a second time point, and determining the quantitative abundance of the MEI border in the second blood sample at the second time point.
27. The method of aspect 25, wherein said monitoring the quantitative abundance of the MEI border adjacent to the oncogene over time comprises obtaining a first tissue sample at a first time point, determining the quantitative abundance of the MEI border in the first tissue sample at the first time point, obtaining a second tissue sample at a second time point, and determining the quantitative abundance of the MEI border in the second tissue sample at the second time point.
28. The method of aspect 27, wherein said first tissue sample and said second tissue sample comprise tumor tissue.
29. The method of any one of aspects 25-28, comprising selecting a treatment to address a cancer related to a defect in the oncogene.
30. The method of aspect 29, comprising administering the treatment to address a cancer related to a defect in the oncogene if the quantitative abundance of the MEI insertion site increases in the sample above a threshold from the first time point to the second time point.
31. The method of aspect 30, wherein the threshold is a 10% increase.
32. The method of aspect 30, wherein the threshold is a 20% increase.
33. The method of aspect 30, wherein the threshold is a 30% increase.
34. The method of aspect 30, wherein the threshold is a 50% increase.
35. The method of aspect 29, comprising administering a first dosage of the treatment to address a cancer related to a defect in the oncogene prior to a first time point, and increasing the dosage if the quantitative abundance of the MEI insertion site fails to decrease in the sample below a threshold from the first time point to the second time point.
36. The method of aspect 35, wherein the threshold is 90% of the first time point amount.
37. The method of aspect 35, wherein the threshold is 80% of the first time point amount.
38. The method of aspect 35, wherein the threshold is 70% of the first time point amount.
39. The method of aspect 35, wherein the threshold is 60% of the first time point amount.
40. The method of aspect 35, wherein the threshold is 50% of the first time point amount.
41. The method of aspect 35, wherein the threshold is 10% of the first time point amount.
42. The method of any one of aspects 28-34, wherein the treatment comprises chemotherapy.
43. The method of any one of aspects 28-34, wherein the treatment comprises radiotherapy.
44. The method of any one of aspects 28-34, wherein the treatment comprises a pharmaceutical that targets a defect in the sequence adjacent to the MEI insertion.
45. The method of any one of aspects 28-34, wherein the treatment comprises a pharmaceutical that targets misregulation of a pathway of which a protein encoded by sequence adjacent to a MEI insertion site participates.
46. The method of any one of aspects 28-34, wherein the treatment comprises a nucleic acid that specifically binds the MEI insertion junction.
47. The method of aspect 42, wherein the nucleic acid comprises a piRNA.
48. The method of aspect 42, wherein the nucleic acid comprises a siRNA.
49. The method of aspect 42, wherein the nucleic acid comprises a CRISPR nucleic acid.
50. The method of aspect 42, wherein the nucleic acid directs methylation of the MEI insertion border.
51. A composition for the in vivo visualization of cancer tissue comprising a nucleic acid probe spanning an MEI border adjacent to an oncogene, coupled to a detection element.
52. The composition of aspect 51, wherein the detection element comprises a fluorophore.
53. The composition of aspect 51, wherein the detection element comprises a photoexcitable moiety.
54. The composition of any one of aspects 51-53, wherein the probe traverses cell membranes.
55. The composition of any one of aspects 51-53, wherein the probe traverses cell nuclear membranes.
56. The composition of any one of aspects 51-53, wherein probe fluorescence is dependent upon probe binding to a target nucleic acid sequence comprising a MEI border adjacent to an oncogene.
57. The composition of any one of aspects 51-56, wherein said probe is visualized by a hand-held fluorophore excitation device.
58. A method for monitoring genomic aging, comprising the steps of quantitatively measuring the number of MEI insertion sites in a first nucleic acid sample at a first time period;
   quantitatively measuring the number of MEI insertion sites in a first nucleic acid sample at a first time period; and
   correlating an increase in MEI insertion borders with an increase in genomic aging.
59. The method of aspect 58, wherein a 10% increase in the number of MEI insertion sites indicates genomic aging.
60. The method of aspect 58, wherein a 20% increase in the number of MEI insertion sites indicates genomic aging.
61. The method of aspect 58, wherein a 30% increase in the number of MEI insertion sites indicates genomic aging.
62. The method of aspect 58, wherein a 50% increase in the number of MEI insertion sites indicates genomic aging.
63. The method of any one of aspects 58-62, comprising recommending an anti-aging regimen if genomic aging is indicated.
64. The method of aspect 63, wherein the anti-aging regimen comprises caloric restriction.
65. The method of aspect 63, wherein the anti-aging regimen comprises administration of an NSAID.
66. The method of aspect 63, wherein the anti-aging regimen comprises administration of a DNA methylase.
67. The method of aspect 63, wherein the anti-aging regimen comprises administration of a reverse-transcriptase inhibitor.
68. The method of aspect 63, wherein the anti-aging regimen comprises administration of a retrovirus inhibitor.
69. The method of aspect 63, wherein the anti-aging regimen comprises administration of an HIV inhibitor.
70. The method of aspect 63, wherein the anti-aging regimen comprises administration of AZT.
71. The method of aspect 63, wherein the anti-aging regimen comprises administration of an HBV inhibitor.
72. The method of aspect 63, wherein the anti-aging regimen comprises administration of ribavirin.
73. The method of aspect 63, wherein the anti-aging regimen comprises administration of a transposase inhibitor.
74. A method for comparing a first nucleic acid sample and a second nucleic acid sample, comprising the steps of
   obtaining Mobile Element Insertion (MEI) border sequence for a plurality of MEI borders of said first nucleic acid sample;
   assaying for the presence of said plurality of MEI borders in said second nucleic acid sample; and
   identifying said second nucleic acid sample as different from said first nucleic acid sample if said second nucleic acid sample lacks an MEI border sequence present in said first nucleic acid sample.
75. The method of aspect 74, comprising identifying said second nucleic acid sample as different from said first nucleic acid sample if said second nucleic acid sample includes an MEI border sequence not present in said first nucleic acid sample.
76. The method of aspect 74, wherein obtaining Mobile Element Insertion (MEI) border sequence for a plurality of MEI borders of said first nucleic acid sample comprises performing whole-genome sequencing of said first nucleic acid sample.
77. The method of aspect 74, wherein obtaining Mobile Element Insertion (MEI) border sequence for a plurality of MEI borders of said first nucleic acid sample comprises performing targeted sequencing of said plurality of MEI borders of said first nucleic acid sample.
78. The method of any one of aspects 74-77, wherein assaying for the presence of said plurality of MEI borders in said second nucleic acid sample comprises performing whole-genome sequencing of said second nucleic acid sample.
79. The method of any one of aspects 74-77, wherein assaying for the presence of said plurality of MEI borders in said second nucleic acid sample comprises performing targeted sequencing of said plurality of MEI borders of said second nucleic acid sample.
80. The method of aspect 79, wherein performing targeted sequencing of said plurality of MEI borders of said second nucleic acid sample comprises contacting said second nucleic acid sample with a panel of primers comprising primers that specifically amplify each MEI insertion site of said first nucleic acid sample.
81. The method of aspect 79, wherein performing targeted sequencing of said plurality of MEI borders of said second nucleic acid sample comprises contacting said second nucleic acid sample with a panel of probes comprising probes that specifically anneal to each MEI insertion site of said first nucleic acid sample.
82. The method of aspect 81, wherein the panel of probes comprises at least one probe bound to a fluorophore such that probe bound to substrate is differentially visualizeable relative to probe not bound to substrate.
83. The method of any one of aspects 74-82, wherein said second sample comprises a forensic sample.
84. The method of any one of aspects 74-82, wherein said second sample comprises a plant sample.
85. The method of aspect 84, wherein said plant sample is a plant crop sample.
86. The method of any one of aspects 74-82, wherein said second sample comprises biohazardous substance.
87. A composition for use in delaying age-related genome deterioration comprising a Mobile Element Insertion inhibiting pharmaceutical.
88. The composition for use of aspect 87, wherein said composition comprises a reverse-transcriptase inhibitor.
89. The composition for use of aspect 87, wherein said composition comprises a retroviral inhibitor.

## Claims

1. A method of measuring mobile element insertions (MEI) comprising the steps of:
(a) obtaining genome sequence information by DNA sequencing from a first biological sample wherein the genome sequence information comprises a plurality of MEI insertion borders;
(b) reviewing the plurality of MEI insertion borders to identify at least one MEI border of interest;
(c) determining a first quantitative abundance of the at least one MEI border of interest in the first biological sample and a second quantitative abundance of the at least one MEI border of interest in a second biological sample; and
(d) determining a difference between the first quantitative abundance and the second quantitative abundance.

2. The method of claim 1, wherein the first biological sample and the second biological sample are derived from one individual.

3. The method of claim 1 or claim 2, wherein the first biological sample and the second biological sample comprise tumor nucleic acids.

4. The method of claim 1 or claim 2, wherein the at least one MEI border of interest comprises a gene related to disease.

5. The method of claim 4, wherein the disease is cancer or aging.

6. The method of claim 4 or claim 5, comprising identifying an individual as likely to benefit from a treatment of the disease.

7. The method of any one of claims 1 to 6, wherein the first biological sample is obtained at a time point before the second biological sample is obtained.

8. The method of any one of claims 1 to 7, wherein the first biological sample or the second biological sample comprise cell-free nucleic acids.

9. The method of any one of claims 1 to 8, wherein (c) comprises targeted sequencing of the at least one MEI border of interest.

10. The method of any one of claims 1 to 8, wherein (c) comprises contacting the first biological sample and the second biological sample to a probe that binds to the at least one MEI border of interest.

11. The method of any one of claims 1 to 8, wherein (c) comprises quantitative PCR with primers that specifically amplify the at least one MEI border of interest.

12. The method of claim 1 or claim 2, wherein the first biological sample or the second biological sample comprises a forensic sample

13. The method of claim 1 or claim 2, wherein the first biological sample or the second biological sample comprises plant tissue.

14. The method of any one of claims 1 to 13, wherein the MEI comprises an L1, an Alu, a SINE, a LINE, anSVA, a type I retrotransposon, or an endogenous retrovirus.

15. The method of any one of claims 1 to 14, wherein the at least one MEI border of interest comprises a promoter region or a gene coding region of a genome.
